(19) Europäisches Patentamt
European Patent Office
Office européen des brevets

(11) **EP 4 596 525 A1**

(12) **EUROPEAN PATENT APPLICATION**
published in accordance with Art. 153(4) EPC

(43) Date of publication:
06.08.2025 Bulletin 2025/32

(21) Application number: 23871425.7

(22) Date of filing: 27.07.2023

(51) International Patent Classification (IPC):
*C07C 6/04* $^{(2006.01)}$   *B01D 3/14* $^{(2006.01)}$
*B01J 8/02* $^{(2006.01)}$   *B01J 29/40* $^{(2006.01)}$
*B01J 29/44* $^{(2006.01)}$   *C07B 61/00* $^{(2006.01)}$
*C07C 1/24* $^{(2006.01)}$   *C07C 11/06* $^{(2006.01)}$

(52) Cooperative Patent Classification (CPC):
(C-Sets available)
**C07C 1/24; B01D 3/14; B01J 8/02; B01J 29/40;
B01J 29/44; C07B 61/00; C07C 1/20; C07C 6/04;
C07C 11/06;** C07C 2521/08; C07C 2529/40;
C07C 2529/44                          (Cont.)

(86) International application number:
**PCT/JP2023/027601**

(87) International publication number:
**WO 2024/070182 (04.04.2024 Gazette 2024/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 30.09.2022  JP 2022157839
30.09.2022  JP 2022157843
30.09.2022  JP 2022158148

(71) Applicant: Asahi Kasei Kabushiki Kaisha
Tokyo 1000006 (JP)

(72) Inventors:
• URAYAMA, Teppei
Tokyo 100-0006 (JP)
• MIYAZAKI, Ryusuke
Tokyo 100-0006 (JP)

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **ETHANOL CONVERSION METHOD, HYDROCARBON PRODUCTION METHOD, PROPYLENE PRODUCTION METHOD, AROMATIC COMPOUND PRODUCTION METHOD, AND ETHANOL CONVERSION DEVICE**

(57)   Provided is a method for converting ethanol, comprising:
a dehydration step of subjecting a dehydration raw material containing ethanol to dehydration reaction by a dehydration catalyst in a reactor to obtain a dehydration reaction gas containing ethylene; and
an olefin conversion step of contacting a raw material mixture containing the dehydration reaction gas with an olefin conversion catalyst in a reactor to obtain a reaction gas containing an olefin having 3 or more carbon atoms, wherein
the raw material mixture contains an ether, and
in the raw material mixture, a value represented by the following expression (1):

$$\frac{EY}{EtOH} - 10 \times \frac{Ether}{EtOH+EY+Ether} \cdots (1)$$

wherein EY represents a molar quantity of ethylene contained in the raw material mixture, EtOH represents a molar quantity of ethanol contained in the raw material mixture, and Ether represents a molar quantity of the ether contained in the raw material mixture
is 0.20 to 4.0.

EP 4 596 525 A1

[Figure 1]

(52) Cooperative Patent Classification (CPC): (Cont.)

C-Sets
C07C 1/20, C07C 11/06;
C07C 1/20, C07C 15/02;
C07C 1/24, C07C 11/04;
C07C 6/04, C07C 11/06;
C07C 6/04, C07C 15/02

## Description

## Technical Field

The present invention relates to a method for converting ethanol, a method for producing a hydrocarbon, a method for producing propylene, a method for producing an aromatic compound, and an apparatus for converting ethanol.

## Background Art

Lower olefins and aromatic compounds are core raw materials important for the chemical industry. Particularly, various methods for producing propylene have been actively developed or modified in anticipation of growing demand therefor. Among them, a method including contacting naphtha or olefins with a catalyst containing zeolite as an active species is known as a general method for producing propylene.

Until now, production of chemical products such as lower olefins or aromatic compounds by using biomass-derived alcohols as raw materials, in addition to olefins, has received attention due to a rising demand for environmental protection in recent years. Particularly, ethanol is a compound whose production method has been established from biomass raw materials. Therefore, early development of an efficient method for converting ethanol has been expected.

For example, Patent Literature 1 discloses a method for converting an olefin or an alcohol. Patent Literature 2 discloses pentasil zeolite as a catalyst. Patent Literature 3 discloses zeolite-supported zinc cerium oxide as a catalyst. For example, Patent Literature 4 discloses a method for producing a lower olefin by using an oxygenate and an olefin having 4 or more carbon atoms as raw materials. Patent Literature 5 discloses a method for producing a lower olefin by using a lower alcohol and naphtha as raw materials.

## Citation List

## Patent Literature

Patent Literature 1: International Publication No. WO2014/025021
Patent Literature 2: International Publication No. WO2015/029355
Patent Literature 3: Chinese Patent Application Publication No. 110560155
Patent Literature 4: U.S. Patent Application Publication No. 2014/0018593
Patent Literature 5: Chinese Patent Application Publication No. 110871107

## Summary of Invention

## Technical Problem

The production of hydrocarbons such as propylene or aromatic compounds by converting ethanol is considered to go through dehydration reaction of ethanol to ethylene. In the conversion of olefins from ethanol, the dehydration reaction of ethanol occurs as described above. The dehydration reaction is endothermic reaction and therefore has the difficulty in adjusting an internal temperature of a reactor due to a decreased temperature of the reactor and may require applying a lot of heat to the reactor. Decrease in reaction temperature reduces the yield of a target light olefin. On the other hand, ethylene conversion to propylene or an aromatic compound is exothermic reaction and may therefore reduce the yield of a target compound by increasing the amount of by-products such as coke, due to an elevated internal temperature of the reactor.

Patent Literatures 1 to 3 each disclose a technique of converting each raw material to a target olefin using a zeolite catalyst. These techniques increase endotherm and exotherm and therefore have the difficulty in controlling temperatures.

Patent Literatures 4 and 5 each disclose a thermal neutralization technique using exothermic reaction by using an oxygen-containing compound such as an alcohol as a raw material. Reaction that induces endothermic reaction by using ethanol as a raw material has the difficulty in controlling temperatures.

Thus, there is a demand for a method for easily controlling an internal temperature of a reactor, particularly, in an olefin conversion step in which temperature fluctuation tends to occur.

Accordingly, an object of the present invention is to provide a method for converting ethanol which easily controls an internal temperature of a reactor and converts ethanol to a target compound in good yield, a method for producing a hydrocarbon, a method for producing propylene, a method for producing an aromatic compound, and an apparatus for converting ethanol.

**Solution to Problem**

The present inventors have conducted diligent studies to attain the object described above, and consequently found that in producing an olefin having 3 or more carbon atoms from ethanol, ethanol can be converted to a target compound in good yield while an internal temperature of a reactor is easily controlled, by involving a dehydration step of subjecting ethanol to dehydration reaction and an olefin conversion step, wherein the raw material mixture contains an ether, and in the raw material mixture, a value represented by the expression (1) falls within a predetermined range.

Specifically, the present invention encompasses the following embodiments.

[1] A method for converting ethanol, comprising:

a dehydration step of subjecting a dehydration raw material containing ethanol to dehydration reaction by a dehydration catalyst in a reactor to obtain a dehydration reaction gas containing ethylene; and
an olefin conversion step of contacting a raw material mixture containing the dehydration reaction gas with an olefin conversion catalyst in a reactor to obtain a reaction gas containing an olefin having 3 or more carbon atoms, wherein
the raw material mixture contains an ether, and
in the raw material mixture, a value represented by the following expression (1):

$$\frac{EY}{EtOH} - 10 \times \frac{Ether}{EtOH + EY + Ether} \cdots \ (1)$$

wherein EY represents a molar quantity of ethylene contained in the raw material mixture, EtOH represents a molar quantity of ethanol contained in the raw material mixture, and Ether represents a molar quantity of the ether contained in the raw material mixture
is 0.20 to 4.0.

[2] The method for converting ethanol according to [1], wherein the value represented by the expression (1) in the raw material mixture is 0.40 to 3.0.
[3] The method for converting ethanol according to [1] or [2], wherein the ether comprises at least one selected from the group consisting of diethyl ether, methyl tertiary butyl ether, and ethyl tertiary butyl ether.
[4] The method for converting ethanol according to any of [1] to [3], wherein a content of the ether in the raw material mixture is 20% by mass or less.
[5] A method for converting ethanol, comprising:

a dehydration step of subjecting a dehydration raw material containing ethanol to dehydration reaction by a dehydration catalyst in an amount of 10 parts by mass or more with respect to 100 parts by mass of the ethanol fed per hour in a reactor to obtain a dehydration reaction gas containing ethylene; and
an olefin conversion step of contacting a raw material mixture containing the dehydration reaction gas with an olefin conversion catalyst in a reactor to obtain a reaction gas containing an olefin having 3 or more carbon atoms.

[6] The method for converting ethanol according to [5], wherein in the dehydration step, a conversion rate of ethanol in the dehydration raw material is brought to 90% by mol or more.
[7] A method for converting ethanol, comprising:

a dehydration step of subjecting a dehydration raw material containing ethanol to dehydration reaction by a dehydration catalyst in a reactor and bringing a conversion rate of ethanol in the dehydration raw material to 90% by mol or more to obtain a dehydration reaction gas containing ethylene; and
an olefin conversion step of feeding ethanol and the dehydration reaction gas into a reactor and contacting the raw material mixture with an olefin conversion catalyst in the reactor to obtain a reaction gas containing an olefin having 3 or more carbon atoms.

[8] The method for converting ethanol according to [7], wherein a weight ratio E1/E2 of ethanol (E1) to be fed into the reactor in the dehydration step to ethanol (E2) to be fed into the reactor in the olefin conversion step is 0.10 to 4.0.
[9] The method for converting ethanol according to any of [1] to [8], wherein a content of propylene in the dehydration reaction gas is 20% by mass or less.

[10] The method for converting ethanol according to any of [1] to [9], wherein a content of diethyl ether in the raw material mixture is 10% by mass or less.

[11] The method for converting ethanol according to any of [1] to [10], wherein the raw material mixture contains a hydrocarbon having 4 to 6 carbon atoms.

[12] The method for converting ethanol according to any of [1] to [11], wherein a molar ratio of olefin having 4 to 6 carbon atoms/ethylene in the raw material mixture is 3.5 or less.

[13] The method for converting ethanol according to any of [1] to [12], wherein the reactor in the olefin conversion step is an adiabatic reactor.

[14] The method for converting ethanol according to [13], wherein the adiabatic reactor in the olefin conversion step is a fixed-bed single-stage adiabatic reactor.

[15] The method for converting ethanol according to any of [1] to [14], further comprising

a separation step of separating the reaction gas into fraction A mainly comprising a hydrocarbon having 1 to 3 carbon atoms and fraction B mainly comprising a hydrocarbon having 4 to 6 carbon atoms.

[16] The method for converting ethanol according to any of [1] to [15], further comprising:

a C1 removal step of separating the fraction A into fraction C mainly comprising a hydrocarbon having 1 carbon atom and fraction D mainly comprising a hydrocarbon having 2 to 3 carbon atoms:

a C2 removal step of separating the fraction D into fraction E mainly comprising a hydrocarbon having 2 carbon atoms and fraction F mainly comprising a hydrocarbon having 3 carbon atoms;
an ethylene purification step of separating the fraction E into fraction G mainly comprising ethylene and fraction H mainly comprising ethane; and
a propylene purification step of separating the fraction F into fraction I mainly comprising propylene and fraction J mainly comprising propane.

[17] The method for converting ethanol according to any of [1] to [16], wherein the dehydration catalyst and/or the olefin conversion catalyst is a solid acidic catalyst.

[18] The method for converting ethanol according to [17], wherein the solid acidic catalyst is a zeolite-containing catalyst.

[19] The method for converting ethanol according to [17] or [18], wherein a silica/alumina molar ratio of zeolite in the zeolite-containing catalyst is 20 to 2000.

[20] The method for converting ethanol according to any of [17] to [19], wherein the zeolite-containing catalyst comprises at least one element selected from the group consisting of phosphorus and an element belonging to group 11 of the periodic table.

[21] A method for producing a hydrocarbon, comprising:

a dehydration step of subjecting a dehydration raw material containing ethanol to dehydration reaction by a dehydration catalyst in a reactor to obtain a dehydration reaction gas containing ethylene; and
an olefin conversion step of contacting a raw material mixture containing the dehydration reaction gas with an olefin conversion catalyst in a reactor to obtain a reaction gas containing an olefin having 3 or more carbon atoms, wherein
the raw material mixture contains an ether, and
in the raw material mixture, a value represented by the following expression (1):

$$\frac{EY}{EtOH} - 10 \times \frac{Ether}{EtOH+EY+Ether} \cdots \ (1)$$

wherein EY represents a molar quantity of ethylene contained in the raw material mixture, EtOH represents a molar quantity of ethanol contained in the raw material mixture, and Ether represents a molar quantity of the ether contained in the raw material mixture
is 0.20 to 4.0.

[22] A method for producing propylene, comprising

a propylene-separating step of separating a fraction mainly comprising propylene from a reaction gas obtained by the method for converting ethanol according to any of [1] to [21].

[23] A method for producing an aromatic compound, comprising

an aromatic compound-separating step of separating a fraction mainly comprising an aromatic compound from a reaction gas obtained by the method for converting ethanol according to any of [1] to [21].

[24] An apparatus for converting ethanol, comprising:

a reactor which subjects a dehydration raw material containing ethanol to dehydration reaction by a dehydration catalyst to obtain a dehydration reaction gas containing ethylene; and
a reactor which contacts a raw material mixture containing the dehydration reaction gas with an olefin conversion catalyst to obtain a reaction gas containing an olefin having 3 or more carbon atoms.

[25] The apparatus for converting ethanol according to [24], further comprising
a separation apparatus which separates the reaction gas into fraction A mainly comprising a hydrocarbon having 1 to 3 carbon atoms and fraction B mainly comprising a hydrocarbon having 4 to 6 carbon atoms.

[26] The apparatus for converting ethanol according to [24] or [25], further comprising:

an ethanol feed line which introduces ethanol to the reactor for obtaining the reaction gas; and
a dehydration reaction gas feed line which introduces the dehydration reaction gas to the reactor for obtaining the reaction gas.

**Advantageous Effects of Invention**

The present invention can provide a method for converting ethanol which easily controls an internal temperature of a reactor and converts ethanol to a target compound in good yield, a method for producing a hydrocarbon, a method for producing propylene, a method for producing an aromatic compound, and an apparatus for converting ethanol.

**Brief Description of Drawings**

[Figure 1] Figure 1 shows a schematic view of one embodiment of an apparatus for converting ethanol.
[Figure 2] Figure 2 shows a schematic view of one embodiment of a fixed-bed single-stage adiabatic reactor.
[Figure 3] Figure 3 shows a schematic view of one embodiment of a single-tube fixed-bed isothermal reactor.
[Figure 4] Figure 4 shows a schematic view of one embodiment of an apparatus for converting ethanol.
[Figure 5] Figure 5 shows a schematic view of one embodiment of an apparatus for converting ethanol.
[Figure 6] Figure 6 shows a schematic view of one embodiment of an apparatus for converting ethanol.

**Description of Embodiments**

Hereinafter, the present invention will be specifically described. The present invention is not limited by the following embodiments (present embodiment) and can be carried out through various changes or modification without departing from the spirit of the present invention.

Herein, a numerical range represented by using "to" means a range including the numerical values described before and after "to" as the minimum value and the maximum value, respectively. In numerical ranges described in stages herein, the upper limit value or the lower limit value of a numerical range at a certain stage may be arbitrarily combined with the upper limit value or the lower limit value of a numerical range at a different stage.

[Method for converting ethanol]

The method for converting ethanol according to the first embodiment comprises:

a dehydration step of subjecting a dehydration raw material containing ethanol to dehydration reaction by a dehydration catalyst in a reactor to obtain a dehydration reaction gas containing ethylene; and
an olefin conversion step of contacting a raw material mixture containing the dehydration reaction gas with an olefin conversion catalyst in a reactor to obtain a reaction gas containing an olefin having 3 or more carbon atoms, wherein the raw material mixture contains an ether, and
in the raw material mixture, a value represented by the following expression (1):

$$\frac{EY}{EtOH} - 10 \times \frac{Ether}{EtOH + EY + Ether} \cdots \ (1)$$

wherein EY represents a molar flow rate of ethylene contained in the raw material mixture, EtOH represents a molar flow rate of ethanol contained in the raw material mixture, and Ether represents a molar flow rate of the ether contained in the raw material mixture

is 0.20 to 4.0.

The first member (EY/EtOH) in the expression (1) represents the molar ratio of ethylene to ethanol in the raw material mixture. In the present embodiment, for example, ethylene, ethanol, and ether involve large heat of reaction for chemical conversion. Among them, the main raw materials ethylene and ethanol in the olefin conversion step have content ratios that largely influence change in internal temperature of a reactor. Accordingly, the amplitude of exotherm based on endotherm in the olefin conversion step has been expressed in the first member by using ethylene which exhibits the exotherm in association with conversion as a numerator and ethanol which exhibits the endotherm in association with conversion as a denominator. However, the first member alone cannot take into consideration the influence of the ether conversion. The quantity of heat absorbed in the conversion reaction of an ether to an olefin is larger than that for ethanol. Therefore, the second member has been established by intending correction for reflecting the influence of the quantity of heat absorbed by the ether on the total heat of reaction.

The second member in the expression (1) is the abundance ratio of the ether to ethylene, ethanol, and ether multiplied by a coefficient. This ratio refers to the molar ratio of ether in the raw material mixture. Therefore, the influence of endothermic reaction by the olefin conversion of ether on the heat of reaction in olefin conversion reaction may be appropriately corrected by multiplying this ratio by an appropriate coefficient and subtracting the obtained value from the first member. On this assumption, the coefficient has been empirically determined through experiments.

For example, in the case of producing 2 mol of propylene from 3 mol of ethanol, 10 kJ of endotherm is involved per mol of propylene. On the other hand, for example, 28 kJ per mol of propylene occurs for using chain diethyl ether having one ether group in the molecule as a raw material, and 33 kJ per mol of propylene occurs for using methyl tertiary butyl ether as a raw material. These raw materials more largely influence the internal temperature of a reactor than that for ethanol as a raw material. As a result of conducting diligent studies to express such influence of ether on the internal temperature of a reactor, a member in which the abundance ratio of ether to ethylene, ethanol, and ether is multiplied by "10" has been conceived in an empirical rule.

In short, the expression (1) represents a value obtained by subtracting the second member which represents the influence of endotherm by ether contained in the raw material mixture from the first member which represents the influence of relative exotherm in a reactor in the olefin conversion step. Thus, the balance between exotherm and endotherm of reaction in the olefin conversion step can be adjusted by setting, to a predetermined range, the value of the expression (1) which represents the amplitude of the influence of endotherm and exotherm in the raw material mixture.

The present embodiment described above can provide a method for converting ethanol which easily controls an internal temperature of a reactor and converts ethanol to a target compound in good yield, a method for producing a hydrocarbon, a method for producing propylene, a method for producing an aromatic compound, and an apparatus for converting ethanol. In short, a dehydration raw material containing ethanol is subjected to dehydration reaction in the dehydration step to prepare ethylene, and the raw material containing ethylene is used in the olefin conversion step, whereby the olefin conversion step can be carried out while a value represented by the expression (1) in the raw material mixture is adjusted to a predetermined range; the balance between exotherm and endotherm of reaction in the olefin conversion step can be adjusted; an internal temperature of a reactor is easily controlled by suppressing temperature fluctuation; and ethanol can be converted to a target compound in good yield. Furthermore, for example, ethylene produced by the dehydration step is fed to olefin conversion, whereby an internal temperature of a reactor is easily controlled even when a raw material containing ether is used; and ethanol can be converted to a target compound in good yield.

The value of the expression (1) is preferably 0.4 to 3.0. When the value of the expression (1) is 0.4 or more, reaction temperature balance easily becomes a positive and the yield of a target compound easily increases. When the value of the expression (1) is 3.0 or less, the maximum value of a reaction temperature is not too high and reduction in performance by coke production can be suppressed, which are preferred. The value of the expression (1) is 0.40 to 3.0, preferably 0.50 to 2.5, further preferably 0.60 to 1.5, in view of easily controlling an internal temperature of a reactor.

The method for converting ethanol according to the second embodiment comprises:

a dehydration step of subjecting a dehydration raw material containing ethanol to dehydration reaction by a dehydration catalyst in an amount of 10 parts by mass or more with respect to 100 parts by mass of the ethanol fed per hour in a reactor to obtain a dehydration reaction gas containing ethylene; and

an olefin conversion step of contacting a raw material mixture containing the dehydration reaction gas with an olefin conversion catalyst in a reactor to obtain a reaction gas containing an olefin having 3 or more carbon atoms.

As mentioned above, there is a demand for a method for easily controlling an internal temperature of a reactor, particularly, in an olefin conversion step in which temperature fluctuation tends to occur. It is considered that the internal temperature of a reactor in the olefin conversion step is easily controlled by subjecting ethanol to dehydration reaction in an established dehydration step for ethanol dehydration reaction, and using a dehydration reaction gas containing ethylene in the olefin conversion step. However, the following problem has been found: in the dehydration step, diethyl ether is produced as a by-product through the dehydration reaction of ethanol, and this diethyl ether exhibits large endotherm in the olefin conversion step and makes it difficult to control the temperature of the olefin conversion step.

Accordingly, an object of the second embodiment is to provide a method for converting ethanol which reduces the amount of diethyl ether produced as a by-product in a dehydration step, a method for producing a hydrocarbon, a method for producing propylene, a method for producing an aromatic compound, and an apparatus for converting ethanol.

The second embodiment described above can provide a method for converting ethanol which reduces the amount of diethyl ether produced as a by-product in a dehydration step, a method for producing a hydrocarbon, a method for producing propylene, a method for producing an aromatic compound, and an apparatus for converting ethanol. It has been found that in the dehydration step, the production of diethyl ether as a by-product is suppressed by using a dehydration catalyst in an amount as large as 10 parts by mass or more with respect to 100 parts by mass of the ethanol fed per hour. This diethyl ether exhibits large endotherm when treated in the olefin conversion step. In short, use of the dehydration catalyst in excess suppresses the production of diethyl ether as a by-product in the dehydration step and therefore facilitates controlling an internal temperature of a reactor in the olefin conversion step.

The second embodiment described above can also provide a method for converting ethanol which easily controls an internal temperature of a reactor by comprising the dehydration step and the olefin conversion step mentioned above, and converts ethanol to a target compound in good yield, a method for producing a hydrocarbon, a method for producing propylene, a method for producing an aromatic compound, and an apparatus for converting ethanol. In short, a dehydration raw material containing ethanol is subjected to dehydration reaction in the dehydration step to prepare ethylene, and the raw material containing ethylene is used in the olefin conversion step, whereby the olefin conversion step can be carried out while the ratio between ethylene and ethanol in the raw material mixture is adjusted to a predetermined range; the balance between exotherm and endotherm of reaction in the olefin conversion step can be adjusted; an internal temperature of a reactor is easily controlled by suppressing temperature fluctuation; and ethanol can be converted to a target compound in good yield.

In the method for converting ethanol according to the second embodiment, the conversion rate of ethanol in the dehydration raw material in the dehydration step is preferably 90% by mol or more.

According to the embodiment as mentioned above, ethanol can be converted to a target olefin by appropriately controlling a reaction temperature in the olefin conversion step, while the influence of the second member in the expression (1) is minimized by suppressing the production of ether as a by-product in the dehydration step. In the second embodiment, the object to control a temperature in the olefin conversion step is attained by enhancing reaction efficiency in the dehydration step and suppressing the production of diethyl ether as a by-product.

The method for converting ethanol according to the third embodiment comprises:

a dehydration step of subjecting a dehydration raw material containing ethanol to dehydration reaction by a dehydration catalyst in a reactor and bringing a conversion rate of ethanol in the dehydration raw material to 90% by mol or more to obtain a dehydration reaction gas containing ethylene; and

an olefin conversion step of feeding ethanol and the dehydration reaction gas into a reactor and contacting the raw material mixture with an olefin conversion catalyst in the reactor to obtain a reaction gas containing an olefin having 3 or more carbon atoms.

As mentioned above, there is a demand for a method for easily controlling an internal temperature of a reactor, particularly, in an olefin conversion step in which temperature fluctuation tends to occur. An object of the third embodiment is to provide a method for converting ethanol which easily controls an internal temperature of a reactor in an olefin conversion step and converts ethanol to a target compound in good yield, a method for producing a hydrocarbon, a method for producing propylene, a method for producing an aromatic compound, and an apparatus for converting ethanol.

The third embodiment can provide a method for converting ethanol which easily controls an internal temperature of a reactor in an olefin conversion step and converts ethanol to a target compound in good yield, a method for producing a hydrocarbon, a method for producing propylene, a method for producing an aromatic compound, and an apparatus for converting ethanol. In short, a dehydration raw material containing ethanol is subjected to dehydration reaction in the dehydration step to prepare ethylene, and the raw material containing ethylene is used in the olefin conversion step, whereby the olefin conversion step can be carried out while the ratio between ethylene and ethanol in the raw material mixture is controlled; the balance between exotherm and endotherm of reaction in the olefin conversion step can be adjusted; an internal temperature of a reactor is easily controlled by suppressing temperature fluctuation; and ethanol can be converted to a target compound in good yield.

In addition, in the dehydration step, the conversion rate of ethanol in the dehydration raw material is brought to 90% by mol or more, whereby the amount of ethylene fed in the olefin conversion step is stabilized; the ratio between ethylene and ethanol is adjusted by feeding ethanol in the olefin conversion step; and an internal temperature of a reactor is easily controlled by suppressing temperature fluctuation. In short, the dehydration step and the olefin conversion step are connected so as to stabilize the ratio between ethylene and ethanol, whereby the ratio of ethylene in the dehydration reaction gas is unlikely to vary even due to time-dependent change such as catalyst deterioration; and an internal temperature of a reactor is easily controlled.

In the third embodiment, the weight ratio E1/E2 of ethanol (E1) to be fed into the reactor in the dehydration step to ethanol (E2) to be fed into the reactor in the olefin conversion step is preferably 0.10 to 4.0, more preferably 0.30 to 3.50, further preferably 0.50 to 3.0. Within this range, an internal temperature of a reactor can be more easily controlled.

The influence of ether on heat of reaction may be reduced by directly controlling the composition of the raw material mixture in the olefin conversion step, for example, by directly feeding ethanol to the olefin conversion step without the dehydration step. In the third embodiment, the object to control a temperature in the olefin conversion step is attained by controlling the ratio of ethanol to be fed in the dehydration step to ethanol to be fed in the olefin conversion step.

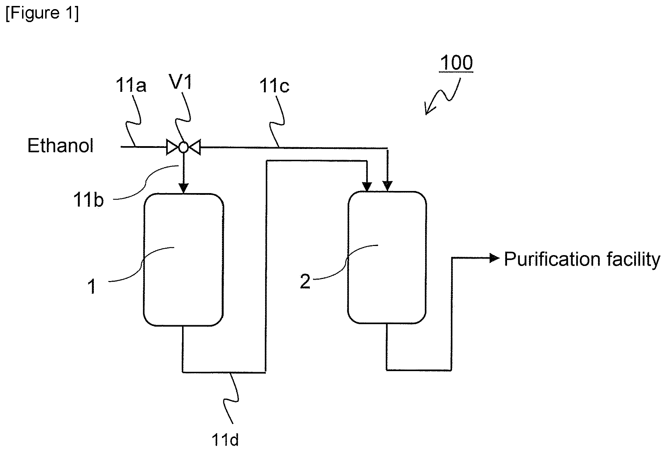

In the third embodiment, a production apparatus provided with a line for directly introducing ethanol to the olefin conversion step is preferably used. As described in Figure 1 mentioned later, dehydration raw material feed line 11b and ethanol feed line 11c for direct introduction to the olefin conversion step are provided in ethanol feed line 11a via control valve V1. The control valve V1 is capable of adjusting the amount of ethanol introduced to reactor 1 and the amount of ethanol introduced to reactor 2 through a bypass.

Hereinafter, a common embodiment among the first embodiment, the second embodiment, and the third embodiment will be described. First, an apparatus for converting ethanol used in the present embodiment will be described, and each step will be further described in order.

Figure 1 is a schematic view of the configuration of apparatus 100 for converting ethanol. The apparatus 100 for converting ethanol has reactor 1 for obtaining a dehydration reaction gas and reactor 2 for obtaining a reaction gas. In the reactor 1, a dehydration raw material containing ethanol is subjected to dehydration reaction by a dehydration catalyst to obtain a dehydration reaction gas containing ethylene. In short, the dehydration step is performed in the reactor 1. The details of the reactor will be described later. In the reactor 2, a raw material mixture containing the dehydration reaction gas is contacted with an olefin conversion catalyst to obtain a reaction gas containing an olefin having 3 or more carbon atoms. In short, the olefin conversion step is performed in the reactor 2. The details of the reactor will be described later.

In the apparatus 100 for converting ethanol, ethanol feed line 11a has dehydration raw material feed line 11b and ethanol feed line 11c for direct introduction to the olefin conversion step via control valve V1. The control valve V1 adjusts the amount of ethanol introduced to the reactor 1 and the amount of ethanol introduced to the reactor 2 through a bypass. The whole amount of ethanol may be subjected to the dehydration step without any bypass.

The dehydration reaction gas obtained from the reactor 1 is introduced to the reactor 2 via dehydration reaction gas feed line 11d. On the other hand, ethanol is introduced from the ethanol feed line 11c to the reactor 2. A mixture of the dehydration reaction gas, ether, and ethanol is converted as a raw material mixture in the reactor 2 in the olefin conversion step. The reaction gas produced in the reactor 2 is sent to a purification facility to obtain a target compound such as propylene or an aromatic compound. Although not shown in the drawing, ether may be introduced from ether feed line 11e to the reactor 2. According to the aspect in which ether is fed from the ether feed line, ether other than ether produced as a by-product from ethanol may be used as a raw material mixture. Surplus ether, if present, is effective, regardless of whether or not to be biomass, because this ether can be used as a raw material in reaction for producing a highly usable olefin.

<Dehydration step>

In the dehydration step according to the present embodiment, a dehydration raw material containing ethanol is subjected to dehydration reaction by a dehydration catalyst in a reactor to obtain a dehydration reaction gas containing ethylene. In this step, $H_2O$ is eliminated from ethanol to obtain ethylene.

(Dehydration raw material)

The raw material to be introduced into the reactor in the dehydration step is referred to as a "dehydration raw material". The dehydration raw material contains ethanol. Ethanol is preferably derived from biomass in view of excellent environmental friendliness. The biomass refers to an organic resource other than a fossil resource originating from an animal or a plant. The term "derived from biomass" refers to a compound produced by using the biomass as a raw material.

The dehydration raw material may contain a hydrocarbon having 4 or more carbon atoms in view of excellent variability of a raw material ratio. The hydrocarbon having 4 or more carbon atoms has lower reactivity than that of ethanol under conditions of the dehydration step. Hence, the contained hydrocarbon having 4 or more carbon atoms can prevent decrease in temperature by heat retained by the hydrocarbon having 4 or more carbon atoms even if heat in the system is absorbed through endothermic reaction. Thus, dehydration reaction can proceed efficiently in the reactor.

Such ethanol contains water as a by-product of the production process or absorbs moisture in atmosphere in its production process. Thus, in the conversion method of the present embodiment, the dehydration raw material ethanol may contain water.

In the dehydration raw material, the mass ratio of hydrocarbon having 4 or more carbon atoms/ethanol is preferably 0.0 to 8.0, more preferably 0.1 to 6.0, further preferably 0.5 to 5.0, still further preferably 0.8 to 3.0, in view of controlling an internal temperature of a reactor and obtaining ethylene through the dehydration reaction of ethanol.

The dehydration raw material may contain ether. However, at least a portion of ether reacts in the dehydration step. Therefore, if a raw material mixture for olefin conversion reaction is intended to contain ether, ether is not added as a raw material in the dehydration step but it is preferred to add ether after the dehydration step or to produce ether from ethanol in the dehydration step, in view of controlling the ratio of ether in the raw material mixture. Examples of the ether include dimethyl ether, diethyl ether, methyl tertiary butyl ether, ethyl tertiary butyl ether, tetrahydrofuran, and tetrahydropyran. Among them, methyl tertiary butyl ether and ethyl tertiary butyl ether are preferred in view of excellent atom efficiency. Ether is preferably derived from biomass in view of excellent environmental friendliness.

The content of ether in the dehydration raw material is preferably 20% by mass or less, more preferably 10% by mass or less, further preferably 5% by mass or less, in view of ethylene production efficiency.

(Reactor)

The reactor for use in the dehydration step (reactor 1 in Figure 1) may be an adiabatic reactor or an isothermal reactor, and a reactor having any mode can be used. Among them, an isothermal reactor is preferably used in view of excellent controllability of an internal temperature of the reactor. An adiabatic reactor is preferably used in view of excellent operability. If necessary, a heating apparatus for heating the raw material can be provided upstream of the reactor.

[Adiabatic reactor]

For the adiabatic reactor, see the description of Adiabatic Fixed-Bed Reactors (Elsevier, 2014, Ch. 1, P. 4, L. 5-24, ISBN: 978-0-12-801306-9). Examples of the adiabatic reactor include fixed-bed adiabatic reactors, moving-bed adiabatic reactors, and fluidized-bed adiabatic reactors. In the method of the present embodiment, a fixed-bed adiabatic reactor is preferred. The fixed-bed adiabatic reactor is more preferably a fixed-bed single-stage adiabatic reactor, which has a single stage of a fixed catalyst bed. Since a carbonaceous material (coke) accumulates on a catalyst in association with reaction, a multi-column switchable fixed-bed single-stage adiabatic reactor, which is capable of removing this carbonaceous material by combustion while continuing the reaction, is preferred.

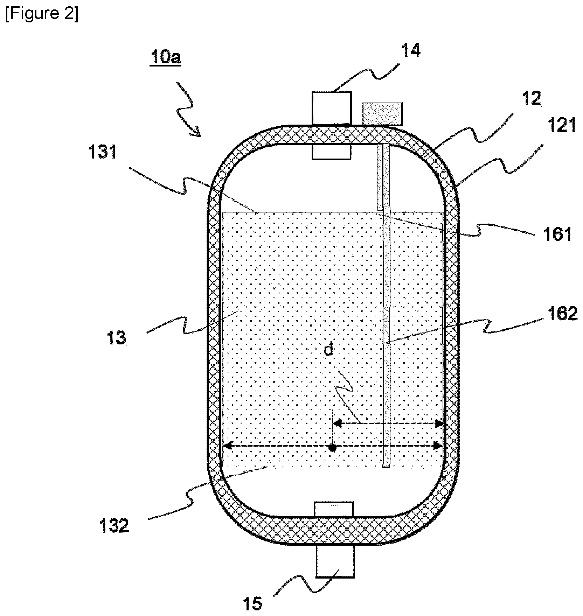

Figure 2 is a schematic view of the configuration of the fixed-bed single-stage adiabatic reactor. Fixed-bed single-stage adiabatic reactor 10a has reaction housing 12 having heat insulation material 121 provided on its outer periphery, catalyst bed 13, reactor inlet 14, and reactor outlet 15. The reaction housing 12, which has the heat insulation material 121 provided on its outer periphery, does not allow the internal heat of the reactor to escape to the outside. In the production method according to the present embodiment, the internal temperature of the reactor can be controlled through exothermic and endothermic reactions.

The catalyst bed 13 is filled with a catalyst mentioned later. First sheathed thermocouple 161 is disposed immediately upstream of catalyst bed inlet 131 of the catalyst bed 13. Second sheathed thermocouple 162 is disposed immediately downstream of catalyst bed outlet 132 of the catalyst bed 3. These thermocouples measure the temperatures of the raw material mixture immediately before contact with the catalyst bed inlet 131 and the reaction gas immediately after passage through the catalyst bed outlet 132. The positions of these thermocouples may be changed, if necessary. The catalyst bed 13 may be of multi-column type and is preferably of single-stage type as shown in Figure 2.

In the fixed-bed single-stage adiabatic reactor 10a, the raw material mixture is introduced from the reactor inlet 14 and contacted with the catalyst bed 13, and the reaction gas is taken out of the reactor outlet 15.

[Isothermal reactor]

The isothermal reactor is a reactor having a function of keeping an internal temperature of the reactor constant by cooling or heating from the outside. Examples of the isothermal reactor include fixed-bed isothermal reactors, moving-bed isothermal reactors, and fluidized-bed isothermal reactors. In the method of the present embodiment, a fixed-bed isothermal reactor is preferred. The fixed-bed isothermal reactor is more preferably a multi-tube fixed-bed isothermal reactor having a smaller size per reactor than that of a single-tube fixed-bed isothermal reactor, in view of excellent controllability of a reaction temperature. Since a carbonaceous material (coke) accumulates on a catalyst in association with reaction, a multi-column switchable fixed-bed isothermal reactor, which is capable of removing this carbonaceous material by combustion while continuing the reaction is preferred.

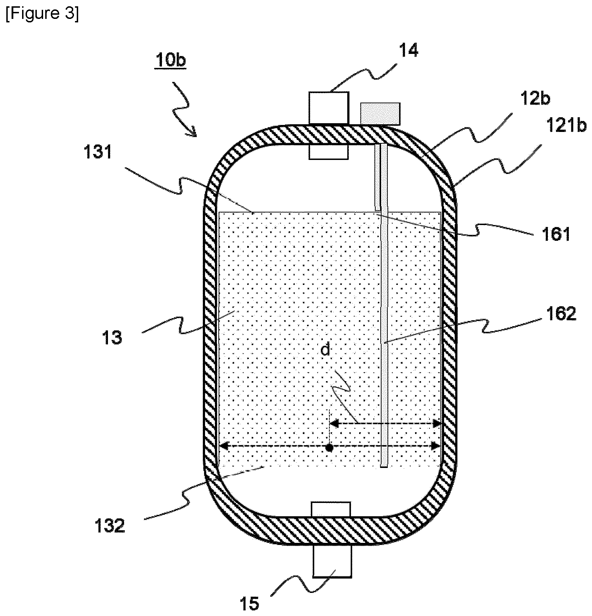

Figure 3 is a schematic view of the configuration of a single-tube fixed-bed isothermal reactor. Single-tube fixed-bed isothermal reactor 10b basically has a common configuration with the fixed-bed single-stage adiabatic reactor 10a. Therefore, the same symbols will be used to designate the same or similar components as those therein, so that the description will be omitted. The fixed-bed single-stage isothermal reactor 10b differs from the fixed-bed single-stage adiabatic reactor 1 in that reaction housing 12b having heat medium layer 121b provided on its outer periphery is used. The reaction housing 12b is heated or cooled by the temperature control of the heat medium layer 121b provided on its outer periphery, and can keep the temperature of catalyst bed 13 constant.

[Reaction conditions]

The reaction temperature in the dehydration step is preferably 200 to 600°C, more preferably 250 to 500°C, further preferably 270 to 480°C, in view of allowing the dehydration reaction of ethanol to proceed.

In the case of using an adiabatic reactor, the reaction temperature is as follows. The catalyst bed inlet temperature is the temperature of the raw material mixture immediately before the raw material fluid comes into contact with the catalyst bed filled into the adiabatic reactor. The catalyst bed outlet temperature is the temperature of the reaction gas immediately after the reaction gas passes through the catalyst bed. In this context, the temperatures of the raw material mixture and the reaction gas refer to temperatures at 0 d to 0.8 d, wherein d represents the distance from the center of the reactor to the inner wall surface of the reactor when the center of the reactor in a plane perpendicular to the flow direction of a fluid is defined as 0. The inlet-outlet average reaction temperature (hereinafter, also simply referred to as a "reaction temperature") is defined as a value calculated according to the expression: [Catalyst bed inlet temperature + Catalyst bed outlet temperature] / 2, wherein the catalyst bed inlet temperature and the catalyst bed outlet temperature are measured, as shown in Figure 2. In the case of using an adiabatic reactor, the catalyst bed inlet temperature is preferably 350°C to 550°C, and the catalyst bed outlet temperature is preferably 100°C to 450°C. Among them, the outlet temperature is more preferably 250°C or higher in view of excellent dehydration reaction efficiency.

In the case of using an isothermal reactor, the internal temperature of the reactor is considered to be uniform. Therefore, the catalyst bed inlet temperature is treated as a dehydration temperature. The catalyst bed inlet temperature is the temperature of the dehydration raw material immediately before the raw material fluid comes into contact with the catalyst bed filled into the reactor. In this context, the temperature of the dehydration raw material refers to a temperature at 0 d to 0.8 d, wherein d represents the distance from the center of the reactor to the inner wall surface of the reactor when the center of the reactor in a plane perpendicular to the flow direction of a fluid is defined as 0. In the transformation method of the present embodiment, the dehydration temperature is preferably 200°C to 450°C. Among them, the temperature is more preferably 350°C or lower in view of excellent deterioration resistance of a heat medium for use in the isothermal reactor.

The reaction pressure in the dehydration step is preferably 0.01 to 3.0 MPaG, more preferably 0.01 to 1.0 MPaG.

The feed rate of the dehydration raw material is preferably 0.1 to 1000 $hr^{-1}$, more preferably 0.1 to 500 $hr^{-1}$, further preferably 0.5 to 100 $hr^{-1}$, further preferably 0.5 to 10 $hr^{-1}$, in terms of the weight hourly space velocity (WHSV) of a dehydration catalyst. WHSV of 10 $hr^{-1}$ or less is particularly preferred in view of being able to suppress the production of diethyl ether as a by-product in the dehydration step. In the conversion method of the present embodiment, WHSV is calculated by converting ethanol to ethylene as shown in the expression given below. The mass flow rate of the raw material fed is preferably 1 kg/hr or more, more preferably 10 kg/hr or more, further preferably 1 t/hr or more, in view of excellent productivity of a target compound.

WHSV ($hr^{-1}$) = Flow rate of ethanol fed in terms of ethylene (kg/hr)/Amount (kg) of catalyst Ethanol flow rate in terms of ethylene (kg/hr) = Ethanol flow rate (kg/hr) $\times$ Molecular weight (g/mol) of ethylene/Molecular weight (g/mol) of ethanol

[Dehydration catalyst]

The dehydration catalyst of the present embodiment is a catalyst that exhibits the ability to catalyze the conversion of ethanol to ethylene through dehydration reaction. The dehydration catalyst is preferably a solid acidic catalyst. Examples of such a catalyst include zeolite-containing catalysts, heteropoly acid catalysts, and alumina catalysts. Examples of the alumina catalyst include SynDol catalysts. Among them, a zeolite-containing catalyst is preferred in view of excellent thermal durability of the catalyst. In the case of using the same type of zeolite as a catalyst in the olefin conversion step and the dehydration step, reduction in the types of by-products produced through both the steps can be expected as compared with the case of using different catalysts, because the properties of catalytic active sites are unified.

(Zeolite-containing catalyst)

The zeolite-containing catalyst is a catalyst powder or compact containing zeolite as an active species. In the conversion method according to the present embodiment, so-called medium pore-size zeolite, which has a pore size of 5 to 6 angstroms, is preferably used as zeolite in the zeolite-containing catalyst described above. The medium pore-size zeolite means "zeolite whose pore size range is in between the pore size of small pore-size zeolite typified by type A zeolite and the pore size of large pore-size zeolite typified by mordenite or type X or Y zeolite". The "medium pore-size zeolite" has a so-called oxygen 10-membered ring in its crystal structure.

Examples of the medium pore-size zeolite include ZSM-5, ZSM-8, ZSM-11, ZSM-12, ZSM-21, ZSM-23, ZSM-35, and ZSM-38. Among them, MFI-type zeolite is preferred, and ZSM-5 is more preferred. Alternatively, zeolite similar to ZSM-5 or ZSM-11 described in Stud. Surf. Sci. Catal. 1987, 33, 167-215 can be used. Among them, MFI-type zeolite is preferred, and ZSM-5 is more preferred, in view of excellent catalyst performance (catalytic activity and durability against coking).

The silica/alumina ($SiO_2/Al_2O_3$) molar ratio of zeolite contained in the zeolite-containing catalyst of the present embodiment can be appropriately selected and is preferably 20 to 2000, more preferably 40 to 1800, and further preferably 150 to 1500 in view of excellent catalytic activity and propylene selectivity. The silica/alumina molar ratio of zeolite can be measured by a known method and can be determined, for example, by completely dissolving the zeolite in an aqueous alkali solution, and analyzing the resulting solution by plasma emission spectroscopy or the like.

A method for synthesizing the zeolite of the present embodiment is not particularly limited, and the zeolite can be produced by optimizing various conditions for a conventionally known hydrothermal synthesis method of MFI-type zeolite. In general, an approach of efficiently obtaining MFI-type zeolite by a hydrothermal synthesis method includes a method including hydrothermally synthesizing the zeolite with an appropriate organic agent (SDA) such as an ammonium salt, a urea compound, an amine, or an alcohol, and a method including hydrothermally synthesizing the zeolite by adding hydrothermally synthesized MFI zeolite as seed crystals or as a seed slurry at a crystalline stage. Not only organic SDA but an inorganic cation or anion is known to be structurally involved, and the zeolite synthesis depends on complex work of individual components. The hydrothermal synthesis method of MFI-type zeolite as mentioned above can produce a suitable catalyst by appropriately optimizing synthesis conditions such as the type of a raw material or an additive (SDA), the amount of the additive, pH, a silica/alumina molar ratio, a medium, raw material charging composition (e.g., the abundance ratio of a cation or an anion), a synthesis temperature, and a synthesis time.

Specific examples thereof include a synthesis method involving use of a seed slurry described in Japanese Patent No. 5426983, and methods illustrated in The Hydrothermal Synthesis of Zeolites (Chemical Reviews, 2003, 103, 663-702).

Commercially available zeolite may be used as long as the zeolite is the aforementioned MFI zeolite having specific physical properties and composition.

The zeolite-containing catalyst according to the present embodiment preferably comprises at least one element selected from the group consisting of phosphorus and an element belonging to group 11 of the periodic table (hereinafter, these elements are collectively referred to as dope elements). Among them, the zeolite-containing catalyst for use as the dehydration catalyst preferably contains phosphorus.

Examples of the form of the phosphorus include phosphorus polymers (e.g., polyphosphoric acid), phosphorus oxides (e.g., $P_2O_5$), and compounds having phosphorus added to aluminum of zeolite. A plurality thereof may be contained. Phosphorus is effective for suppressing dealumination of zeolite when the zeolite contains aluminum. In the method according to the present embodiment, water may be produced in a reactor through dehydration reaction of an alcohol and contained in the raw material ethanol. Therefore, a high-temperature water vapor atmosphere, which causes dealumination, is easily created in the reactor. Dealumination causes deterioration of activity by the structural collapse of the zeolite-containing catalyst. However, the dealumination of zeolite is suppressed and the durability of the catalyst is enhanced, when the zeolite-containing catalyst contains phosphorus.

Examples of the element belonging to group 11 of the periodic table include copper, silver, and gold. The dealumination of zeolite is suppressed and the durability of the catalyst is enhanced, when the zeolite-containing catalyst contains the element belonging to group 11 of the periodic table. The element belonging to group 11 of the periodic table is preferably silver in view of excellent supporting efficiency.

The content of the dope element contained in the zeolite-containing catalyst is preferably 0.01 to 2.0% by mass based on the mass of the whole catalyst, and is more preferably 0.05 to 2.0% by mass in view of the excellent effect of suppressing dealumination.

In the present embodiment, the content of the dope element in the zeolite-containing catalyst refers to a value found using an X-ray fluorescence analyzer. The content of phosphorus, copper, silver, or gold can be measured using a commercially available X-ray fluorescence analyzer under usual conditions in accordance with its instruction manual. In the case of using, for example, trade name "RIX3000" manufactured by Rigaku Corp., measurement conditions can involve a tube voltage of 50 kV and a tube current of 50 mA using P-K$\alpha$ ray.

In the present embodiment, phosphoric acid and/or phosphate (hereinafter, also referred to as a "phosphorus raw material") is used as a raw material for the phosphorus contained in the zeolite-containing catalyst. The phosphorus raw material is more preferably phosphate, and the phosphate is more preferably a compound having a solubility of 1 g or more in 100 g of water at 25°C.

Examples of the phosphoric acid include phosphoric acid and pyrophosphoric acid. Examples of the phosphate include phosphoric acid ammonium salts such as ammonium phosphate, diammonium hydrogen phosphate, ammonium dihydrogen phosphate, and ammonium sodium hydrogen phosphate, potassium hydrogen phosphate, aluminum hydrogen phosphate, sodium phosphate, and potassium phosphate. Among them, a phosphoric acid ammonium salt having a relatively high solubility in water is preferred, and at least one selected from the group consisting of ammonium phosphate, diammonium hydrogen phosphate, and ammonium dihydrogen phosphate is more preferred. These compounds may each be used singly or may be used in combination of two or more.

In the present embodiment, a nitric acid metal salt such as copper nitrate or silver nitrate may be used as a raw material for the element belonging to group 11 of the periodic table contained in the zeolite-containing catalyst. A zeolite-containing catalyst containing sodium as a counter cation is used in ion exchange with the nitric acid metal salt and the resultant is sintered to obtain a zeolite-containing catalyst containing an element belonging to group 11 of the periodic table.

The zeolite-containing catalyst of the present embodiment can be produced by using the aforementioned zeolite having specific physical properties and composition, and molding the zeolite, for example, as described below. The molding method is not particularly limited, and a general method can be used. Specific examples thereof include a method including compression-molding a catalytic component, a method including extrusion-molding a catalytic component, and a spray dry molding method optimal for a fluidized-bed reaction scheme.

A binder can be used in the molding. The binder is not particularly limited, and, for example, silica, alumina, and kaolin can each be used alone or can be used as a mixture. Commercially available products can be used as these binders. The mass ratio of zeolite/binder is preferably in the range of 10/90 to 90/10, more preferably in the range of 20/80 to 80/20. Among them, a silica binder is preferably used in view of excellent coking resistance.

In the method for converting ethanol according to the present embodiment, the zeolite-containing catalyst may be subjected to a pretreatment step, prior to contacting the raw material with the zeolite-containing catalyst. The pretreatment step is preferably a heat treatment step at a temperature of 300°C or higher in the presence of water vapor. The pretreatment tends to more remarkably produce the effect of suppressing the deterioration of the catalyst or improving selectivity. In the case of the method described above, the treatment is preferably performed at a temperature of 300°C or higher and 900°C or lower in an atmosphere of, but not particularly limited to, a mixed gas of air or an inert gas (such as nitrogen) and steam (water vapor) circulated under conditions involving a water vapor partial pressure of 0.01 atm or more. The heat treatment temperature is more preferably a temperature of 400°C or higher and 700°C or lower. This pretreatment step can be performed using a reactor for converting ethanol and ethylene.

A dehydration reaction gas containing ethylene is obtained by the dehydration step described above.

The content of ethylene in the dehydration reaction gas is preferably 20 to 80% by mass, more preferably 25 to 70% by mass, further preferably 25 to 60% by mass.

The content of water in the dehydration reaction gas is preferably 20 to 80% by mass, more preferably 25 to 70% by mass, further preferably 25 to 60% by mass.

The dehydration reaction gas may contain ether. The content of ether is preferably 40% by mass or less, more preferably 30% by mass or less, further preferably 20% by mass or less in view of the production efficiency of a target compound.

The dehydration reaction gas may contain diethyl ether. However, it is preferred to not contain diethyl ether.

The content of diethyl ether in the dehydration reaction gas is preferably 10% by mass or less, more preferably 5% by mass or less, further preferably 1% by mass or less.

The dehydration reaction gas may contain a hydrocarbon having 4 or more carbon atoms. The contained hydrocarbon having 4 or more carbon atoms facilitates adjusting a reaction temperature in the dehydration step.

The content of the hydrocarbon having 4 or more carbon atoms in the dehydration reaction gas is preferably 10 to 60% by mass, more preferably 20 to 55% by mass, further preferably 30 to 50% by mass.

The conversion rate of ethanol in the dehydration step is preferably 80% by mol or more, more preferably 85% by mol or more, further preferably 90% by mol or more.

<Olefin conversion step>

In the olefin conversion step according to the present embodiment, a raw material mixture containing the dehydration reaction gas is contacted with an olefin conversion catalyst in a reactor to obtain a reaction gas containing an olefin having 3 or more carbon atoms. Ethanol is dehydrated in the dehydration step, and the resultant is used in the olefin conversion step. As a result, a component ratio in the raw material mixture is adjusted, and an internal temperature of a reactor is easily controlled.

Preferably, the raw material mixture for use in the olefin conversion step according to the present embodiment contains ether, and a value represented by the expression (1) is 0.20 to 4.0. When the compositional ratio of the raw material mixture falls within this range, the balance between endothermic reaction and exothermic reaction in the reactor is good and the internal temperature of the reactor is easily controlled. Ethanol and ether involve endotherm in a process of conversion to an olefin having 3 or more carbon atoms in the olefin conversion step. On the other hand, ethylene involves exotherm in a process of conversion to an olefin having 3 or more carbon atoms in the olefin conversion step. Particularly, ether has a larger quantity of heat absorbed than that of ethanol. Therefore, in the expression mentioned above, the ratio of ether is expressed as a member different from the ethylene/ethanol molar ratio and multiplied by "10". Thus, thermal neutralization has presumably been able to be achieved by combining endothermic reaction and exothermic reaction that do not inhibit each other. In short, the conversion reaction of ethylene to propylene and the conversion reaction of ethanol and ether to propylene have been found to proceed without inhibiting each other, and the combination of the exothermic reaction and the endothermic reaction has presumably enabled a reaction temperature to be controlled in the present embodiment. However, the cause is not limited thereto.

The value represented by the expression (1) is preferably 0.40 to 3.0, more preferably 0.50 to 2.5, further preferably 0.6 to 1.5, in view of easily controlling an internal temperature of a reactor.

In the expression (1), Ether represents a molar quantity of ether contained in the raw material mixture. The ether is preferably ether having 10 or less carbon atoms, more preferably ether having 8 or less carbon atoms, further preferably ether having 6 or less carbon atoms, in view of excellent reactivity of olefin conversion reaction. The ether preferably contains one ether group in view of easy control of heat of reaction (quantity of heat absorbed that is not too large). Examples of such ether include dimethyl ether, diethyl ether, methyl tertiary butyl ether, ethyl tertiary butyl ether, tetrahydrofuran, and tetrahydropyran. The ether in the expression (1) may be diethyl ether, methyl tertiary butyl ether, or ethyl tertiary butyl ether.

The ethylene/(ethanol + 2 × diethyl ether) molar ratio of the raw material mixture (hereinafter, also simply referred to as a "raw material mixture compositional ratio") for use in the olefin conversion step may be 0.20 to 4.0. When the raw material mixture compositional ratio falls within this range, the balance between endothermic reaction and exothermic reaction in the reactor is good and the internal temperature of the reactor is easily controlled. Ethanol and diethyl ether involve endotherm in a process of conversion to an olefin having 3 or more carbon atoms in the olefin conversion step. On the other hand, ethylene involves exotherm in a process of conversion to an olefin having 3 or more carbon atoms in the olefin conversion step. Particularly, diethyl ether involves endothermic reaction of two atoms in the olefin conversion step. Therefore, in the expression mentioned above, the ratio of diethyl ether is multiplied by "2". On the other hand, ethylene involves exotherm in a process of conversion to an olefin having 3 or more carbon atoms in the olefin conversion step. Thus, thermal neutralization has presumably been able to be achieved by combining endothermic reaction and exothermic reaction that do not inhibit each other. In short, the conversion reaction of ethylene to propylene and the conversion reaction of ethanol and ether to propylene have been found to proceed without inhibiting each other, and the combination of the exothermic reaction and the endothermic reaction has presumably enabled a reaction temperature to be controlled in the present embodiment. However, the cause is not limited thereto.

The present inventors have studied the control of heat of reaction in a method for converting ethanol, comprising: a dehydration step of subjecting a dehydration raw material containing ethanol to dehydration reaction by a dehydration catalyst in a reactor to obtain a dehydration reaction gas containing ethylene; and an olefin conversion step of contacting a raw material mixture containing the dehydration reaction gas with an olefin conversion catalyst in a reactor to obtain a reaction gas containing an olefin having 3 or more carbon atoms. Specifically, it has been found that when the ethylene/(ethanol + 2 × diethyl ether) molar ratio of the raw material mixture is adjusted to 0.20 to 4.0 in light of the quantity of heat generated and the quantity of heat absorbed through reaction from the raw material to a product in the olefin conversion step, there are tendencies to suppress temperature fluctuation, easily control an internal temperature of a reactor, and be able to convert ethanol to a target compound in good yield.

In short, the finding has been gained that a dehydration raw material containing ethanol is subjected to dehydration reaction in the dehydration step to prepare ethylene, and the raw material containing ethylene is used in the olefin conversion step, whereby the olefin conversion step can be carried out while the ethylene/(ethanol + 2 × diethyl ether) molar ratio of the raw material mixture is adjusted; the balance between exotherm and endotherm of reaction in the olefin conversion step can thereby be adjusted.

Furthermore, studies have been made on enhancing the accuracy of control of heat of reaction while expanding an application range, when the raw material mixture contains ether other than diethyl ether. As a result, the modified expression (1) has been conceived.

The ethylene/(ethanol + 2 × diethyl ether) molar ratio is preferably 0.20 to 4.0, more preferably 0.30 to 3.5, further preferably 0.5 to 3.0, in view of easily controlling an internal temperature of a reactor.

In the present embodiment, the weight ratio E1/E2 of ethanol (E1) to be fed into the reactor in the dehydration step to ethanol (E2) to be fed into the reactor in the olefin conversion step is preferably 0.10 to 4.0, more preferably 0.30 to 3.50, further preferably 0.50 to 3.0. Within this range, an internal temperature of a reactor can be more easily controlled.

[Raw material mixture]

In the olefin conversion step of the present embodiment, a raw material mixture containing the dehydration reaction gas is used. In the raw material mixture, ethanol may be mixed in addition to the dehydration reaction gas. Ethanol is preferably derived from biomass in view of excellent environmental friendliness. The biomass refers to an organic resource other than a fossil resource originating from an animal or a plant. The term "derived from biomass" refers to a compound produced by using the biomass as a raw material.

Ethylene contained in the raw material mixture is preferably ethylene contained in the dehydration reaction gas. Water is produced as a by-product in a process of converting ethanol to ethylene. Therefore, the dehydration reaction gas generally contains water.

The raw material mixture can further comprise "additional ethylene" in addition to ethylene contained in the dehydration reaction gas, in view of excellent variability of a raw material ratio. Ethylene produced by any of various production methods can be used as the "additional ethylene". For example, ethylene obtained through thermal decomposition of naphtha and/or ethane, direct or oxidative dehydrogenation reaction of ethane, or dehydration reaction of ethanol can be used.

The raw material mixture may contain ether. In the first embodiment, the content thereof is set such that the value of the expression (1) satisfies 0.20 to 4.0. The ether is preferably ether having 10 or less carbon atoms, more preferably ether having 8 or less carbon atoms, further preferably ether having 6 or less carbon atoms, in view of excellent reactivity of olefin conversion reaction. The ether preferably contains one ether group in view of easy control of heat of reaction (quantity of heat absorbed that is not too large). Examples of such ether include dimethyl ether, diethyl ether, methyl tertiary butyl ether, ethyl tertiary butyl ether, tetrahydrofuran, and tetrahydropyran. Among them, methyl tertiary butyl ether and ethyl tertiary butyl ether are preferred in view of excellent atom efficiency. Ether is preferably derived from biomass in view of excellent environmental friendliness. Thus, the ether preferably comprises at least one selected from the group consisting of diethyl ether, methyl tertiary butyl ether, and ethyl tertiary butyl ether.

The raw material mixture may further comprise a hydrocarbon having 4 to 6 carbon atoms. Examples of the hydrocarbon having 4 to 6 carbon atoms include olefins having 4 to 6 carbon atoms, and saturated hydrocarbons having 4 to 6 carbon atoms. Among them, an olefin having 4 to 6 carbon atoms can provide a target compound such as propylene by contact with a conversion catalyst, as in ethylene and ethanol. Examples of the hydrocarbon having 4 to 6 carbon atoms include butene, pentene, hexene, butane, pentane, and hexane. In the raw material mixture, the molar ratio of olefin having 4 to 6 carbon atoms/ethylene is preferably 3.0 or less, more preferably 1.0 or less, further preferably 0.14 to 1.0. The term "olefin" described above includes linear, branched, and cyclic olefins as well as cycloparaffin.

The raw material mixture can comprise a hydrocarbon having 7 or more carbon atoms or an oxygenate other than ethanol, such as methanol, propanol, dimethyl ether, or diethyl ether, in addition to ethylene, ethanol, and the hydrocarbon having 4 to 6 carbon atoms described above. The hydrocarbon having 7 or more carbon atoms or the oxygenate can provide a target compound such as propylene by contact with a conversion catalyst, as in ethylene and ethanol.

The raw material mixture can comprise an inert gas for dilution such as nitrogen, hydrogen, or methane in addition to the above-defined organic matter capable of being converted to propylene by the olefin conversion step. However, it is preferred to not perform dilution with hydrogen. Although hydrogen may be used for suppressing the coking deterioration of a catalyst, hydrogen also causes hydrogenation reaction of produced propylene or the like, which has an adverse effect of reducing propylene purity (propylene / (propylene + propane)) [mol/mol]. The method of the present embodiment has a small coking deterioration rate of a catalyst and permits stable operation even if dilution with hydrogen is not performed. Therefore, it is preferred to not dilute with hydrogen.

The total content of ethylene and ethanol in the raw material mixture is preferably 30 to 100% by mass, more preferably 40 to 100% by mass, further preferably 50 to 100% by mass, based on the total amount of the conversion raw material. However, as for ethanol, the mass thereof in terms of ethylene is used in calculation. When the total content of ethylene and ethanol falls within the range of 50% by mass or more, the heat of reaction is more easily controlled by satisfying the value of the expression (1) in the olefin conversion reaction step.

The content of ether in the raw material mixture is preferably 20% by mass or less, more preferably 10% by mass or less, further preferably 5% by mass or less. The content of ether in the raw material mixture may be 0.01% by mass or more, may be 0.05% by mass or more, or may be 0.2% by mass or more. When the content of ether falls within the range of 20% by mass or less, the heat of reaction is more easily controlled by satisfying the value of the expression (1) in the olefin conversion reaction step.

The total content of olefins having 4 to 6 carbon atoms in the raw material mixture is preferably 65% by mass or less, more preferably 10 to 55% by mass.

The raw material mixture may comprise diethyl ether.

The content of diethyl ether in the raw material mixture is preferably 10% by mass or less, more preferably 5% by mass or less, further preferably 1% by mass or less. The content of diethyl ether in the raw material mixture may be 0.01% by mass or more, may be 0.05% by mass or more, or may be 0.2% by mass or more.

The raw material mixture may contain an olefin having 4 to 6 carbon atoms in view of more easily controlling an internal temperature of a reactor.

The molar ratio of olefin having 4 to 6 carbon atoms/ethylene in the raw material mixture is preferably 3.5 or less, more preferably 3.0 or less, further preferably 1.0 or less, still further preferably 0.14 to 1.0. The term "olefin" described above includes linear, branched, and cyclic olefins as well as cycloparaffin.

In the conversion method of the present embodiment, the raw material mixture can comprise water vapor. Ethylene and ethanol contained in the raw material mixture are produced by various production methods and therefore contain "water vapor generated in the production process". In this context, the "water vapor generated in the production process" refers to moisture that is generated in an ethylene and/or ethanol production process and remains unremoved.

In the method for producing propylene according to the present embodiment, the raw material mixture can comprise water vapor in addition to the "water vapor generated in the production process". The water vapor is effective for decreasing an olefin partial pressure, thereby suppressing coking deterioration and improving the yield of a lower olefin. On the other hand, the water vapor might promote dealumination of zeolite. Therefore, preferably, the raw material mixture comprises no water vapor in addition to the "water vapor generated in the production process".

[Reactor]

The reactor for use in the olefin conversion step (reactor 2 in Figure 1) may be an adiabatic reactor or an isothermal reactor, and a reactor having any mode can be used. Among them, an isothermal reactor is preferably used in view of excellent controllability of an internal temperature of the reactor. An adiabatic reactor is preferably used in view of excellent operability. The adiabatic reactor and the isothermal reactor are as described above.

[Reaction conditions]

The reaction temperature in the olefin conversion step is preferably 300 to 600°C, more preferably 400 to 590°C, further preferably 450 to 550°C, in view of obtaining an olefin having 3 or more carbon atoms in good yield. The reaction temperature is preferably 400°C or higher in view of excellent propylene yield. On the other hand, the reaction temperature is preferably 600°C or lower in view of suppressing the acceleration of coking deterioration, which is promoted at a high temperature.

The reaction temperature in the adiabatic reactor is as defined in the dehydration step.

The temperature difference between the catalyst bed outlet temperature and the catalyst bed inlet temperature is preferably -80 K to 80 K, more preferably -60 K to 60 K.

The reaction pressure is preferably 0.01 to 3.0 MPaG, more preferably 0.01 to 1.0 MPaG.

The feed rate of the raw material mixture is preferably 0.1 to 1000 $hr^{-1}$, more preferably 0.1 to 500 $hr^{-1}$, further preferably 0.5 to 100 $hr^{-1}$, in terms of the weight hourly space velocity (WHSV) of an olefin conversion catalyst. In the olefin conversion step, WHSV is calculated by converting ethanol to ethylene as shown in the expression given below. The mass flow rate of the raw material fed is preferably 1 kg/hr or more, more preferably 10 kg/hr or more, further preferably 1 t/hr or more, in view of excellent productivity of a target compound.

$$\text{WHSV (hr}^{-1}) = \text{Mass flow rate of olefin fed (kg/hr) / Amount (kg) of catalyst}$$

Mass flow rate of olefin fed (kg/hr) = Ethylene flow rate (kg/hr) + Ethanol flow rate in terms of ethylene (kg/hr) + Ether flow rate in terms of the corresponding olefin (kg/hr) + Flow rate (kg/hr) of olefin having 4 to 6 carbon atoms Ethanol flow rate in terms of ethylene (kg/hr) = Ethanol flow rate (kg/hr) × Molecular weight (g/mol) of ethylene/Molecular weight (g/mol) of ethanol Ether flow rate in terms of the corresponding olefin (kg/hr) = Ether flow rate (kg/hr) × (Molecular weight (g/mol) of ether - Molecular weight (g/mol) of water)/ Molecular weight (g/mol) of ether

[Olefin conversion catalyst]

The olefin conversion catalyst is a catalyst that exhibits the ability to catalyze the conversion of an olefin and ethanol to a target compound such as propylene. The olefin conversion catalyst is preferably a solid acidic catalyst. Such a catalyst is preferably a zeolite-containing catalyst in view of excellent thermal durability of the catalyst. The zeolite-containing catalyst is as described above in the dehydration catalyst mentioned above. A common challenge to conventional olefin production involving use of zeolite is coking deterioration caused by a heavy carbonaceous material (coke) that accumulates inside zeolite pores through reaction with a hydrocarbon and deactivates the zeolite. For regenerating catalyst performance, it is necessary to remove coke by combustion in an atmosphere containing an oxygen molecule. In association with this combustion of coke, however, structural collapse of zeolite proceeds to induce permanent deterioration of the catalyst impossible to regenerate. According to the conversion method of the present embodiment, the zeolite-containing catalyst used easily maintains its activity because the production of coke can be suppressed.

In the olefin conversion step, the silica/alumina molar ratio of zeolite in the zeolite-containing catalyst is preferably 20 to 2000, more preferably 40 to 1800, further preferably 150 to 1500, in view of excellent catalytic activity and propylene selectivity.

In the olefin conversion step, the zeolite-containing catalyst preferably comprises at least one element selected from the group consisting of phosphorus and an element belonging to group 11 of the periodic table. Among them, the zeolite-containing catalyst for use as the dehydration catalyst preferably contains phosphorus or silver.

<Regeneration step>

The method for converting ethanol according to the present embodiment may comprise the regeneration step of combusting coke deposited on the catalyst (hereinafter, also referred to as a "regeneration step"). When a catalyst is used in reaction for a long period, coke may accumulate onto the catalyst, causing coking deterioration. If the catalyst has undergone coking deterioration, for example, coke on the catalyst can be removed by combustion at a temperature of 400 to 700°C in air or in a mixed gas of air and/or oxygen and an inert gas preferably under conditions involving, for example, an oxygen concentration of 0.1 to 2.0% by volume to regenerate the catalyst that has undergone coking deterioration. Any regeneration method may be adopted, including extra-reactor regeneration, which involves discharging the catalyst from the reactor and performing regeneration treatment outside the reactor, and intra-reactor regeneration, which involves performing regeneration treatment inside the reactor without discharging the catalyst from the reactor. Alternatively, reaction-regeneration switching operation may be performed by adopting a switchable reactor.

(Reaction-regeneration switching operation)

The reaction-regeneration switching operation is an operational procedure of performing the reaction step and the regeneration step at the same time using a double-column or multi-column switchable reactor. In the case of, for example, a triple-column switchable reactor, two columns are used in the reaction step while the remaining one column is used in the catalyst regeneration. Then, the reaction step of one of the columns used in the reaction step is terminated, and the catalyst regeneration is performed instead. The reaction step is performed in the one column used in the catalyst regeneration. As a result, the catalyst regeneration can be performed while production capacity derived from two columns is maintained. Such a reaction format, also called merry-go-round scheme, is preferred in view of production efficiency because of the absence of the need of discontinuing the production process for catalyst regeneration.

[Product: reaction gas comprising olefin having 3 or more carbon atoms]

In the conversion method according to the present embodiment, the raw material mixture is contacted with the conversion catalyst to obtain a reaction gas comprising an olefin having 3 or more carbon atoms. The reaction gas may comprise ethylene. The reaction gas may comprise hydrogen, an aliphatic hydrocarbon having 1 to 3 carbon atoms, an aliphatic hydrocarbon having 4 to 6 carbon atoms, an aromatic compound, and a hydrocarbon having 9 or more carbon atoms.

Herein, an aliphatic hydrocarbon having 1 to 3 carbon atoms, an aliphatic hydrocarbon having 4 to 6 carbon atoms, an aromatic compound, and a hydrocarbon having 9 or more carbon atoms are referred to as target compounds.

<Separation step>

In the conversion method according to the present embodiment, the target compound can be efficiently purified by establishing a separation step. In the separation step, the reaction gas obtained by the preceding reaction step is separated into fraction A mainly comprising a hydrocarbon having 2 to 3 carbon atoms and fraction B mainly comprising a hydrocarbon having 4 to 6 carbon atoms using a separation apparatus. In this way, each fraction can be separated to efficiently separate the target compound. The separation apparatus for use in the separation step is, for example, a distillation column. Alternatively, the target compound such as ethylene, propylene, or an aromatic compound may be separated from the reaction gas in a purification system of an ethylene plant by introducing the reaction gas obtained by the preceding reaction step to the purification system, without establishing the separation step.

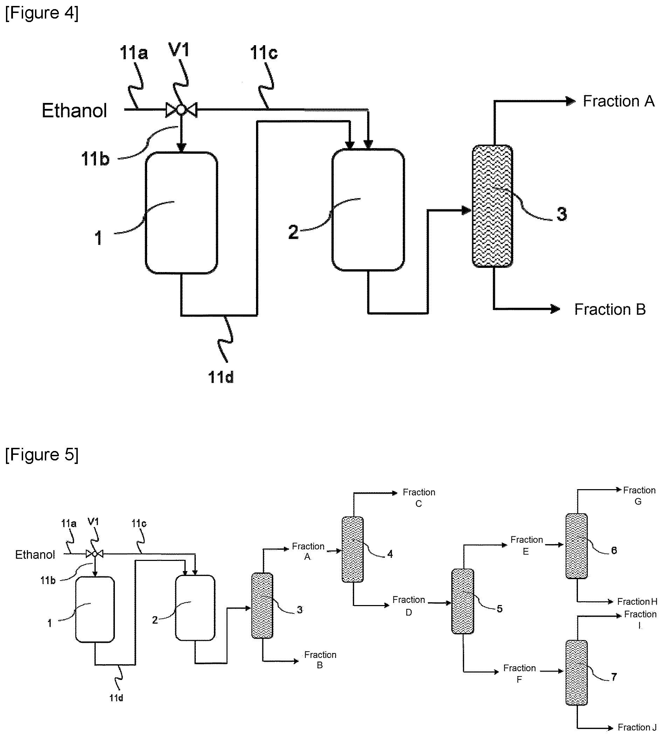

The method according to the present embodiment may be carried out with an apparatus having reactor 1, reactor 2, and first distillation column 3, as shown in Figure 4. The reaction gas obtained by the olefin conversion step performed in the reactor 2 can be separated into fraction A mainly comprising a hydrocarbon having 1 to 3 carbon atoms and fraction B mainly comprising a hydrocarbon having 4 to 6 carbon atoms by the first distillation column 3. The separation of ethylene and propylene from the reaction gas is efficiently performed by separating ethylene from the fraction A by a distillation column (not shown) and further separating propylene by a distillation column (not shown). Although not shown, at least a portion of the reaction gas and/or the fraction A may be introduced to the refining system of an ethylene plant, and a target compound such as ethylene or propylene can be separated from the reaction gas by the refining system.

The method according to the present embodiment can be carried out with an apparatus having reactor 1, reactor 2, first distillation column 3, second distillation column 4, third distillation column 5, fourth distillation column 6, and fifth distillation column 7, as shown in Figure 5. The reaction gas obtained by the olefin conversion step performed in the reactor 2 is separated into fraction A mainly comprising a hydrocarbon having 1 to 3 carbon atoms and fraction B mainly comprising a hydrocarbon having 4 to 6 carbon atoms in the first distillation column 3. The fraction A is separated into fraction C mainly comprising a hydrocarbon having 1 carbon atom and fraction D mainly comprising a hydrocarbon having 2 to 3 carbon atoms in the second distillation column 4. The fraction D is separated into fraction E mainly comprising a hydrocarbon having 2 carbon atoms and fraction F mainly comprising a hydrocarbon having 3 carbon atoms in the third distillation column 5. The fraction E is separated into fraction G mainly comprising ethylene and fraction H mainly comprising ethane in the fourth distillation column 6. The fraction F is separated into fraction I mainly comprising propylene and fraction J mainly comprising propane. In this way, the target compound such as propylene can be efficiently obtained by continuously separating the reaction gas into each fraction. The target compound may be obtained by a similar method after the fraction A is separated into a fraction mainly comprising a hydrocarbon having 1 to 2 carbon atoms and a fraction mainly comprising a hydrocarbon having 3 carbon atoms.

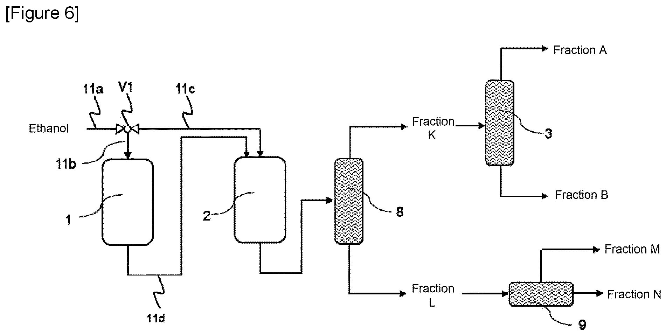

The method according to the present embodiment can be carried out with an apparatus having reactor 1, reactor 2, cooler 8, oily water separator 9, and first distillation column 3, as shown in Figure 6. The reaction gas obtained by the olefin conversion step performed in the reactor 2 is separated into fraction K mainly comprising a hydrocarbon having 1 to 6 carbon atoms and fraction L mainly comprising water, a hydrocarbon having 7 or more carbon atoms, and an aromatic compound in the cooler 8. The phrase "mainly comprising water, a hydrocarbon having 7 or more carbon atoms, and an aromatic compound" in the fraction L means that the total amount of water, the hydrocarbon having 7 or more carbon atoms, and the aromatic compound exceeds 50% by mass of the total amount of the fraction L. The fraction K is separated into fraction A mainly comprising a hydrocarbon having 1 to 3 carbon atoms and fraction B mainly comprising a hydrocarbon having 4 to 6 carbon atoms in the first distillation column 3. The fraction L is separated into fraction M mainly comprising a hydrocarbon having 7 or more carbon atoms and an aromatic compound and fraction N mainly comprising water in the oily water separator 9. The aromatic compound can be efficiently purified by introducing the fraction M to a purification system. In this way, the target compound such as propylene or an aromatic compound can be efficiently purified by separating the reaction gas into fraction A, fraction B, and fraction M. The target compound such as ethylene, propylene, or an aromatic compound may be separated from the reaction gas in a purification system of an ethylene plant by introducing at least a portion of the fraction A, the fraction B, and the fraction M to the purification system.

The term "mainly comprising" for various fractions means that the total mass of the component described with the term "mainly comprising" exceeds 50% by mass based on each fraction. These separation steps can be carried out by combining various known methods such as distillation and extraction.

As mentioned above, various hydrocarbons are obtained by the method for converting ethanol according to the present embodiment. In short, the method for converting ethanol according to the present embodiment is a method for producing a hydrocarbon in another aspect. The hydrocarbon may be any of saturated hydrocarbons and unsaturated hydrocarbons. Examples of the unsaturated hydrocarbon include olefins and aromatic hydrocarbon compounds.


[Method for producing propylene and method for producing aromatic compound]


Various chemical products are obtained by separating target compounds from the reaction gases obtained by the present embodiment.

The method for producing propylene according to the present embodiment comprises a propylene separation step of separating a fraction mainly comprising propylene from a reaction gas obtained by the method for converting ethanol according to the present embodiment. In the propylene separation step, a known propylene purification method such as distillation can be used. The propylene separation step can be carried out by connecting a portion or the whole of the reaction gas to a purification system of an ethylene plant, and using a facility attached to the ethylene plant.

The method for producing an aromatic compound according to the present embodiment comprises an aromatic compound separation step of separating a fraction mainly comprising an aromatic compound from a reaction gas obtained by the method for converting ethanol according to the present embodiment. In the aromatic compound separation step, a known aromatic compound purification method such as distillation can be used. The propylene separation step can be carried out by connecting a portion or the whole of the reaction gas to a purification system of an ethylene plant, and using a facility attached to the ethylene plant. Examples

Hereinafter, the present embodiment will be further specifically described with reference to Examples. However, the present embodiment is not limited by Examples given below.

[Methods for measuring various properties]

Various properties were measured as described below.

(1) Molar ratio of silica/alumina of zeolite in zeolite-containing catalyst

Zeolite was completely dissolved in a sodium hydroxide solution to provide a solution. The amounts of Si and Al contained in the solution were measured by an ordinary method using an ICP (inductively coupled plasma) spectrometry apparatus (manufactured by Rigaku Corp., trade name "JY138"), and the molar ratio of silica/alumina was determined from the results. The measurement conditions were set to high-frequency power: 1 kw, plasma gas: 13 L/min, sheath gas: 0.15 L/min, nebulizer gas: 0.25 L/min, Si measurement wavelength: 251.60 nm, and Al measurement wavelength: 396.152 nm.

(2) Content of dope element in zeolite-containing catalyst

The content of a dope element in a zeolite-containing catalyst was measured by a routine method using an X-ray fluorescence analyzer (manufactured by Rigaku Corp., trade name "RIX3000").

(3) Structural type of zeolite

The structural type of zeolite in a zeolite-containing catalyst was identified by measuring the X-ray diffraction pattern of the zeolite using an X-ray analysis apparatus (manufactured by Rigaku Corp., trade name "RINT"), and referring to the diffraction pattern of known zeolite. The measurement conditions were as follows.

Cu negative electrode
Tube voltage: 40 kV
Tube current: 30 mA
Scan speed: 1 deg/min

[Method for converting ethanol]

(Reactor)

In Examples and Comparative Examples given below, evaluation was conducted using the fixed-bed single-stage adiabatic reactor 1 shown in Figure 2 or the single-tube fixed-bed isothermal reactor shown in Figure 3.

(Temperature measurement)

The temperature of a catalyst bed inlet and the temperature of a catalyst bed outlet were measured by a thermocouple inserted from the outside of the reactor. Specifically, as shown in Figures 2 and 3, temperatures at 0.5d to 0.6d were measured, wherein d represents the distance from the center of the reactor to the inner wall surface of the reactor when the center of the reactor in a plane perpendicular to the flow direction of a fluid is defined as 0. The influence of heat dissipation ascribable to the insertion of this thermocouple is negligibly small. If necessary, the lowest internal temperature of the reactor was measured by moving the thermocouple in the flow direction of a fluid.

(Dehydration raw material)

In Examples and Comparative Examples, ethanol was mixed, if necessary, with a hydrocarbon having 4 or more carbon atoms to prepare a dehydration raw material.

(Evaluation of reaction in dehydration step)

Reaction was carried out in accordance with Examples and Comparative Examples given below. A portion of a gas from the reactor outlet was sampled every 3 hours from the start of the reaction, introduced to a gas chromatograph (TCD and FID detectors), and analyzed for reaction gas composition. The reaction was terminated 48 hours after the start of the reaction. An average value of GC analysis results from the start of the reaction to the termination of the reaction was calculated. In the case of using an adiabatic reactor, the catalyst bed inlet temperature and the catalyst bed outlet temperature were described together. In the case of using an isothermal reactor, the catalyst bed inlet temperature was described as a reaction temperature.

(Raw material mixture)

In Examples given below, a dehydration reaction gas was mixed, if necessary, with a bypass raw material, ether, and a hydrocarbon having 4 or more carbon atoms to prepare a raw material mixture.
The value of the expression (1) was calculated from EY which represents the molar flow rate of ethylene contained in the raw material mixture, EtOH which represents the molar flow rate of ethanol contained in the raw material mixture, and Ether which represents the molar flow rate of ether contained in the raw material mixture.
The ethylene/(ethanol + 2 $\times$ diethyl ether) molar ratio and the molar ratio of olefin having 4 to 6 carbon atoms/ethylene in the raw material mixture gas were calculated according to the following expressions.

Ethylene/(ethanol + 2 $\times$ diethyl ether) molar ratio (-)= Ethylene molar flow rate (mol/hr)/(Ethanol molar flow rate (mol/hr) + 2 $\times$ Diethyl ether molar flow rate (mol/hr)) Molar ratio (-) of olefin having 4 to 6 carbon atoms/ethylene = Flow rate (mol/hr) of olefin having 4 to 6 carbon atoms/ Ethylene molar flow rate (mol/hr)

(Evaluation of reaction in olefin conversion step)

Reaction was carried out in accordance with Examples and Comparative Examples given below such that an inlet-outlet average reaction temperature was 540°C. A portion of a gas from the reactor outlet was sampled every 3 hours from the start of the reaction, introduced to a gas chromatograph (TCD and FID detectors), and analyzed for reaction gas composition. The reaction was terminated 48 hours after the start of the reaction. An average value of GC analysis results from the start of the reaction to the termination of the reaction was calculated. The inlet-outlet average reaction temperature is calculated according to the following expression and described as "Reaction temperature" in the tables.

Inlet-outlet average reaction temperature (°C) = [Catalyst bed inlet temperature (°C) + Catalyst bed outlet temperature (°C)]/2

(Coke yield)

In Examples and Comparative Examples given below, nitrogen was fed into a reactor after the termination of reaction to purge a hydrocarbon, and a catalyst bed was kept at 500°C. Subsequently, air/nitrogen having an oxygen concentration of 2% by volume was circulated thereinto to remove coke on the catalyst by combustion. In this respect, a gas from the reactor outlet was regularly sampled, and the regeneration gas was analyzed using a gas chromatograph to measure $CO_2$ and CO concentrations. From the resulting values, the amount of carbon deposited on the catalyst was determined, and this amount was regarded as the amount of coke. The method for analyzing the regeneration gas using a gas chromatograph will be described below (Analysis conditions for gas chromatograph). The coke yield in Examples and Comparative Examples given below was determined according to the following expression.

Coke yield (wtppm) = Amount of coke/[Flow rate of olefin fed (kg/hr) $\times$ Reaction time (hr)]

Flow rate of olefin fed (kg/hr) = Ethylene flow rate (kg/hr) + Ethanol flow rate in terms of ethylene (kg/hr) + Ether flow rate in terms of the corresponding olefin (kg/hr) + Flow rate (kg/hr) of olefin having 4 to 6 carbon atoms Ethanol flow rate in terms of ethylene (kg/hr) = Ethanol flow rate (kg/hr) $\times$ Molecular weight (g/mol) of ethylene/Molecular weight (g/mol) of ethanol Ether flow rate in terms of the corresponding olefin (kg/hr) = Ether flow rate (kg/hr) × (Molecular weight (g/mol) of ether - Molecular weight (g/mol) of water)/ Molecular weight (g/mol) of ether

[Analysis conditions for gas chromatograph]

(Analysis of regeneration gas)

Apparatus: GC-8A from Shimadzu Corp.
Column: The following columns (1) and (2) were connected in parallel and used.
Column (1): SUS column (inside diameter: 3 mm, length: 3 m) packed with 80-to 100-mesh molecular sieve 5A (manufactured by FUJIFILM Wako Pure Chemical Corp.)
Column (2): 80- to 100-mesh Porapac-Q (inside diameter: 3 mm, length: 2 m) manufactured by Waters Associates (USA) and SUS resistant column (inside diameter: 3 mm, length: 1 m) connected directly
Column temperature: 70°C
Carrier gas (helium) flow rate: 60 mL/min

(Analysis of reaction gas)

Apparatus: GC-2030 manufactured by Shimadzu Corp.
Column: Custom capillary column SPB-1 (inside diameter: 0.25 mm, length: 60 m, film thickness: 3.0 $\mu$m) manufactured by Supelco, Inc. (USA)
Amount of sample gas: 1 mL (a sampling line was kept at 200°C to 300°C)
Heating program: Held at 40°C for 12 minutes, subsequently heated to 200°C at 5°C/min, and then held at 200°C for 22 minutes.
Split ratio: 200:1
Carrier gas (nitrogen) flow rate: 120 mL/min
FID detector: Air feed pressure of 50 kPa (approximately 500 mL/min), hydrogen feed pressure of 60 kPa (approximately 50 mL/min)
Measurement method: A TCD detector and an FID detector were connected in series. Compositional analysis was conducted on the basis of data on hydrogen detected in the TCD detector and data on oxygenates such as hydrocarbons and ethanol detected in the FID detector. The concentration of each component in a reaction gas was determined by use of the calibration curve method, and the mass per hour of the compound produced through reaction was determined.

(Ethanol conversion rate and productivity of diethyl ether as by-product)

The ethanol conversion rate indicates the amount of ethanol converted in the dehydration step, and the productivity of diethyl ether as a by-product indicates the conversion rate of ethanol to diethyl ether. These rates were calculated according to the following expressions.

Ethanol conversion rate (% by mol) = (1 - Amount (kg/hr) of ethanol contained in dehydration reaction gas/Flow rate (kg/hr) of ethanol fed to dehydration step) × 100 Productivity (% by mol) of diethyl ether as by-product = (2 × Amount (kmol/hr) of diethyl ether produced as by-product/Flow rate (kmol/hr) of ethanol fed to dehydration step) × 100

(Propylene yield)

The propylene yield indicates the selectivity for propylene in the olefin conversion step and was calculated according to the following expression.

Propylene yield (% by mass) = Mass per hour of propylene produced in olefin conversion step (kg/hr)/Mass flow rate of olefin fed (kg/hr) Flow rate of olefin fed (kg/hr) = Ethylene flow rate (kg/hr) + Ethanol flow rate in terms of ethylene (kg/hr) + Ether flow rate in terms of the corresponding olefin (kg/hr) + Flow rate (kg/hr) of olefin having 4 to 6 carbon atoms Ethanol flow rate in terms of ethylene (kg/hr) = Ethanol flow rate (kg/hr) $\times$ Molecular weight (g/mol) of ethylene/Molecular weight (g/mol) of ethanol Ether flow rate in terms of the corresponding olefin (kg/hr) = Ether flow rate (kg/hr) $\times$ (Molecular weight (g/mol) of ether - Molecular weight (g/mol) of water)/ Molecular weight (g/mol) of ether

(Aromatic yield)

The aromatic yield indicates the selectivity for an aromatic compound in the olefin conversion step and was calculated according to the following expression.

Aromatic yield (% by mass) = Mass per hour of aromatic compound produced in olefin conversion step (kg/hr)/Flow rate of olefin fed (kg/hr) Flow rate of olefin fed (kg/hr) = Ethylene flow rate (kg/hr) + Ethanol flow rate in terms of ethylene (kg/hr) + Ether flow rate in terms of the corresponding olefin (kg/hr) + Flow rate (kg/hr) of olefin having 4 to 6 carbon atoms Ethanol flow rate in terms of ethylene (kg/hr) = Ethanol flow rate (kg/hr) $\times$ Molecular weight (g/mol) of ethylene/Molecular weight (g/mol) of ethanol Ether flow rate in terms of the corresponding olefin (kg/hr) = Ether flow rate (kg/hr) $\times$ (Molecular weight (g/mol) of ether - Molecular weight (g/mol) of water)/ Molecular weight (g/mol) of ether

[Production Example 1: Preparation of zeolite-containing catalyst 1]

Clay obtained from 80 parts by mass of medium pore-size zeolite proton-type ZSM-5 (silica/alumina molar ratio: 40) and 20 parts by mass of alumina was kneaded, followed by extrusion molding to obtain an extrudate adjusted to a diameter of 1.6 mm and a length of 4 to 6 mm. The obtained extrudate was dried at 350°C for 5 hours and then calcined at 600°C for 5 hours to obtain zeolite-containing catalyst 1.

[Production Example 2: Preparation of zeolite-containing catalyst 2]

Clay obtained from 70 parts by mass of medium pore-size zeolite proton-type ZSM-5 (silica/alumina molar ratio: 1000) and 30 parts by mass of silica (whose moisture content was adjusted using colloidal silica and fumed silica) was kneaded, followed by extrusion molding to obtain an extrudate adjusted to a diameter of 1.6 mm and a length of 4 to 6 mm. The obtained extrudate was dried at 350°C for 5 hours and then calcined at 600°C for 5 hours to obtain zeolite-containing catalyst 2.

[Production example 3: Preparation of zeolite-containing catalyst 3]

Clay obtained from 70 parts by mass of medium pore-size zeolite proton-type ZSM-5 (silica/alumina molar ratio: 280) and 30 parts by mass of silica (whose moisture content was adjusted using colloidal silica and fumed silica) was kneaded, followed by extrusion molding to obtain an extrudate adjusted to a diameter of 1.6 mm and a length of 4 to 6 mm. The obtained extrudate was dried at 350°C for 5 hours and then calcined at 600°C for 5 hours to obtain zeolite-containing catalyst 3.

[Production Example 4: Preparation of zeolite-containing catalyst 4]

Clay obtained from 70 parts by mass of medium pore-size zeolite proton-type ZSM-5 (silica/alumina molar ratio: 280) and 30 parts by mass of silica (whose moisture content was adjusted using colloidal silica and fumed silica) was kneaded, followed by extrusion molding to obtain an extrudate adjusted to a diameter of 1.6 mm and a length of 4 to 6 mm. The obtained extrudate was dried at 350°C for 5 hours, and the obtained dried product was then allowed to support a predetermined amount of an aqueous diammonium hydrogen phosphate solution to obtain a phosphorus-modified product. The obtained phosphorus-modified product was calcined at 600°C for 5 hours in an air atmosphere. The calcined product was filled into a reactor, and a water vapor-nitrogen mixed gas containing 80% by volume of water vapor was fed and circulated thereinto under conditions involving a pressure of 0.1 MPaG and a temperature of 600°C for 24 hours to obtain zeolite-containing catalyst 4. In this respect, the content of a phosphorus element contained in the zeolite-containing catalyst was 0.18% by mass.

[Production Example 5: Preparation of zeolite-containing catalyst 5]

Clay obtained from 70 parts by mass of medium pore-size zeolite proton-type ZSM-5 (silica/alumina molar ratio: 1000) and 30 parts by mass of silica (whose moisture content was adjusted using colloidal silica and fumed silica) was kneaded, followed by extrusion molding to obtain an extrudate adjusted to a diameter of 1.6 mm and a length of 4 to 6 mm. The obtained extrudate was dried at 350°C for 5 hours and then calcined at 600°C for 5 hours to obtain a catalyst precursor. The obtained catalyst precursor was stirred for 1 hour in a 0.1 N aqueous sodium nitrate solution, then filtered, washed, and calcined at 600°C for 5 hours to obtain a sodium-exchanged product. The sodium-exchanged product was stirred for 1 hour in a 0.01 N aqueous silver nitrate solution, subjected to filtration and washing three repeated times, and calcined at 600°C for 5 hours to obtain a silver-exchanged product. A water vapor-air mixed gas containing 80% by volume of water vapor was fed and circulated into the silver-exchanged product under conditions involving a pressure of 0.1 MPaG and a temperature of 600°C for 24 hours to obtain zeolite-containing catalyst 5. In this respect, the content of a silver element contained in the zeolite-containing catalyst was 0.16% by mass.

[Production Example 6: Method for preparing zeolite-containing catalyst 6]

Clay obtained from 70 parts by mass of medium pore-size zeolite proton-type ZSM-5 (silica/alumina molar ratio: 1000) and 30 parts by mass of silica (whose moisture content was adjusted using colloidal silica and fumed silica) was kneaded, followed by extrusion molding to obtain an extrudate adjusted to a diameter of 1.6 mm and a length of 4 to 6 mm. The obtained extrudate was dried at 350°C for 5 hours, and the obtained dried product was then allowed to support a predetermined amount of an aqueous diammonium hydrogen phosphate solution to obtain a phosphorus-modified product. The obtained phosphorus-modified product was calcined at 600°C for 5 hours in an air atmosphere. The calcined product was filled into a reactor, and a water vapor-nitrogen mixed gas containing 80% by volume of water vapor was fed and circulated thereinto under conditions involving a pressure of 0.1 MPaG and a temperature of 600°C for 24 hours to obtain zeolite-containing catalyst 6. In this respect, the content of a phosphorus element contained in the zeolite-containing catalyst was 0.05% by mass.

[Example 1]

(Dehydration step)

Of 10.50 kg/hr in total of 93.3% by mass of ethanol used in Example 1, 6.21 kg/hr (5.80 kg/hr of ethanol + 0.41 kg/hr of water) was used as a dehydration raw material subjected to the dehydration step, and 4.29 kg/hr (4.00 kg/hr of ethanol + 0.29 kg/hr of water) was used as the bypass raw material directly subjected to the olefin conversion step. In the dehydration step, 6.21 kg/hr of ethanol described above and 3.70 kg/hr of the hydrocarbon having 4 or more carbon atoms shown in Table 1 were fed at WHSV of 3.0 and a pressure of 0.20 MPaG to the fixed-bed single-stage adiabatic reactor filled with the zeolite-containing catalyst 1 (silica/alumina molar ratio: 40, alumina binder) to carry out the dehydration step. The fixed-bed single-stage adiabatic reactor had a catalyst bed inlet temperature of 480°C and a catalyst bed outlet temperature of 285°C. The concentration of each component in the obtained dehydration reaction gas is shown in Table 2.

[Table 1]

Table 1

|  | Content ratio (% by mass) |
|---|---|
| Butene | 28.9 |
| Butane | 22.0 |
| Pentene | 14.2 |

(continued)

|  | Content ratio (% by mass) |
|---|---|
| Pentane | 19.4 |
| Hexene | 3.1 |
| Heptene | 0.6 |
| Octene | 0.1 |
| Others | 11.7 |
| Total | 100.0 |

(Preparation of raw material mixture)

The dehydration reaction gas obtained in the dehydration step was mixed with the bypass raw material mentioned above to prepare a raw material mixture. In the raw material mixture, the ethylene/(ethanol + 2 × diethyl ether) molar ratio was 1.06, the value of the expression (1) was 0.96, and the molar ratio of olefin having 4 to 6 carbon atoms/ethylene was 0.26.

(Olefin conversion step)

The raw material mixture was fed at WHSV of 2.0 and a pressure of 0.15 MPaG to the fixed-bed single-stage adiabatic reactor filled with the zeolite-containing catalyst 2 (silica/alumina molar ratio: 1000, silica binder) to carry out the olefin conversion step. After a lapse of 24 h from the start of the reaction, the fixed-bed single-stage adiabatic reactor had a catalyst bed inlet temperature of 534°C, a catalyst bed outlet temperature of 546°C, and a temperature difference of 12 K between the inlet and the outlet. The average propylene yield and the average aromatic yield from the start of the reaction to the termination of the reaction were 20.8% by mass and 2.3% by mass, respectively. The coke yield of the zeolite-containing catalyst 2 after the completion of 48-hour operation was 238 wtppm. The detailed reaction results and reaction conditions are shown in Table 3.

[Examples 2 to 4]

Reaction was performed in the same manner as in Example 1 except that in the dehydration step, the amounts of the dehydration catalyst and the dehydration raw material fed and the amount of the bypass raw material fed were changed as shown in Table 2; and in the olefin conversion step, the olefin conversion catalyst and various conditions were changed as shown in Table 3. The results are shown in Tables 2 and 3.
As seen from Examples 1 to 4, decrease in internal temperature of a reactor was able to be relieved even for the adiabatic reactor by feeding a hydrocarbon having 4 or more carbon atoms in the dehydration step.

[Examples 5 and 6]

Reaction was performed in the same manner as in Example 1 except that in the dehydration step, the single-tube fixed-bed isothermal reactor was used, and the amounts of the dehydration catalyst and the dehydration raw material fed and the amount of the bypass raw material fed were changed as shown in Table 2; and in the olefin conversion step, the olefin conversion catalyst and various conditions were changed as shown in Table 3. The results are shown in Tables 2 and 3.
Since change in internal temperature of a reactor is small in the isothermal reactor, the catalyst bed inlet temperature was used as a reaction temperature. The lowest internal temperature of the reactor is also described in Table 2.
When Examples 1 to 4 were compared with Examples 5 and 6, the value of the expression (1) was 3.0 or less for the composition of the raw material mixtures of Examples 1 to 4. Therefore, temperature elevation in the olefin conversion step can be suppressed, and the coke yield can be kept low. When the value of the expression (1) is 3.0 or less, the amount of coke on the catalyst can be suppressed during reaction and catalyst performance can be maintained during reaction, because of the coke yield kept low.

[Examples 7 and 8]

Reaction was performed in the same manner as in Example 5 except that in the dehydration step, the amounts of the dehydration catalyst and the dehydration raw material fed and the amount of the bypass raw material fed were changed as shown in Table 4; and in the olefin conversion step, the olefin conversion catalyst and various conditions were changed as shown in Table 5. The results are shown in Tables 4 and 5.

[Example 9]

Reaction was performed in the same manner as in Example 1 except that in the dehydration step, the amounts of the dehydration catalyst and the dehydration raw material fed and the amount of the bypass raw material fed were changed as shown in Table 4; and in the olefin conversion step, the olefin conversion catalyst and various conditions were changed as shown in Table 5. The results are shown in Tables 4 and 5.

In Example 9, water was fed in addition to 93.3% by mass of ethanol as the dehydration raw material. Decrease in internal temperature of a reactor was able to be relieved even for the adiabatic reactor by adding water.

[Comparative Example 1]

(Dehydration step)

In Comparative Example 1, 10.50 kg/hr in total of 93.3% by mass of ethanol was used as a dehydration raw material subjected to the dehydration step. The dehydration raw material was fed at WHSV of 4.0 and a pressure of 0.20 MPaG to the fixed-bed single-stage adiabatic reactor filled with the zeolite-containing catalyst 1 (silica/alumina molar ratio: 40, alumina binder) to carry out the dehydration step under a constant condition of 350°C. The concentration of each component in the obtained dehydration reaction gas is shown in Table 4. In this respect, the ethanol conversion rate was 98.2% by mol. The dehydration reaction gas obtained in the dehydration step was used directly as a raw material mixture in the olefin conversion step.

(Olefin conversion step)

The raw material mixture was fed at WHSV of 3.0 and a pressure of 0.15 MPaG to the fixed-bed single-stage adiabatic reactor filled with the zeolite-containing catalyst 2 (silica/alumina molar ratio: 1000, silica binder) to carry out the olefin conversion step. In this respect, the catalyst bed inlet temperature was 419°C, the catalyst bed outlet temperature was 661°C, and the temperature difference between the inlet and the outlet was 242 K. In Comparative Example 1, the activity of the catalyst was drastically reduced as the reaction time passed. After a lapse of 24 hours, the reaction was terminated because the propylene yield and the aromatic yield became 6.3% by mass and 0.5% by mass, respectively. The reaction conditions and the like are shown in Table 5.

The comparison of Examples with Comparative Example 1 revealed that the internal temperature of the adiabatic reactor cannot be controlled by merely connecting the dehydration step to the olefin conversion step and feeding ethanol-derived ethylene in excess based on ethanol and diethyl ether in the olefin conversion step; and this promotes the deterioration of the catalyst, resulting in reduced yield of the target compound.

[Comparative Example 2]

In Comparative Example 2, the whole amount of ethanol was subjected directly to the olefin conversion step without establishing the dehydration step. 93.3% by mass of ethanol was fed at WHSV of 3.0 and a pressure of 0.15 MPaG to the fixed-bed single-stage adiabatic reactor filled with the zeolite-containing catalyst 2 (silica/alumina molar ratio: 1000, silica binder) to carry out the olefin conversion step. In this respect, the catalyst bed inlet temperature was 592°C, the catalyst bed outlet temperature was 488°C, and the temperature difference between the inlet and the outlet was 104 K. The average propylene yield and the average aromatic yield from the start of the reaction to the termination of the reaction were 11.4% by mass and 0.9% by mass, respectively. The coke yield of the zeolite-containing catalyst 2 after the completion of 48-hour operation was 379 wtppm. The detailed reaction results and reaction conditions are shown in Table 5.

The comparison of Examples with Comparative Example 2 revealed that when ethanol is fed directly to the olefin conversion step without establishing the dehydration step, the internal temperature of the adiabatic reactor cannot be controlled due to endothermic reaction, thereby decreasing the reactor outlet temperature, and reducing the yield of the target compound by promoting the deterioration of the catalyst.

[Example 10]

Example 10 was carried out in the same manner as in Example 1 except that ethyl tertiary butyl ether (0.50 kg/hr) was used as the raw material mixture in addition to the dehydration reaction gas and the bypass raw material. The detailed reaction results and reaction conditions are shown in Table 6. The amount of the catalyst filled was increased such that WHSV was compatible with Example 1.

[Example 11]

Example 11 was carried out in the same manner as in Example 1 except that methyl tertiary butyl ether (0.50 kg/hr) was used as the raw material mixture in addition to the dehydration reaction gas and the bypass raw material. The detailed reaction results and reaction conditions are shown in Table 6. The amount of the catalyst filled was increased such that WHSV was compatible with Example 1.

[Example 12]

Example 12 was carried out in the same manner as in Example 6 except that methyl tertiary butyl ether (4.0 kg/hr) was used as the raw material mixture in addition to the dehydration reaction gas and the bypass raw material. The detailed reaction results and reaction conditions are shown in Table 6. The amount of the catalyst filled was increased such that WHSV was compatible with Example 6.

[Comparative Example 3]

Comparative Example 3 was carried out in the same manner as in Example 1 except that methyl tertiary butyl ether (4.0 kg/hr) was used as the raw material mixture in addition to the dehydration reaction gas and the bypass raw material. As a result, the reaction was terminated because the reaction temperature was rapidly decreased in the reactor and the catalyst bed inlet temperature was instable. Unreacted ethanol and methyl tertiary butyl ether were detected at the reactor outlet.

The comparison of Examples with Comparative Example 3 revealed that the internal temperature of the adiabatic reactor is easily controlled by adjusting the value represented by the expression (1) to within a predetermined range in the olefin conversion step.

[Table 2]

Table 2

| | | Example 1 | Example 2 | Example 3 | Example 4 | Example 5 | Example 6 |
|---|---|---|---|---|---|---|---|
| Dehydration catalyst | | 1 | 3 | 4 | 2 | 2 | 2 |
| Silica/alumina ratio | [-] | 40 | 280 | 280 | 1000 | 1000 | 1000 |
| Dope element | [-] | - | - | P | - | - | - |
| Dope element content | [% by mass] | - | - | 0.18% | - | - | - |
| Amount of catalyst | [kg] | 1.23 | 0.81 | 1.55 | 1.69 | 0.99 | 0.99 |
| Total amount of dehydration raw material fed | [kg/hr] | 9.91 | 7.77 | 11.52 | 9.38 | 12.06 | 8.36 |
| Ethanol | [kg/hr] | 5.80 | 3.80 | 7.30 | 5.30 | 7.80 | 7.80 |
| Water | [kg/hr] | 0.41 | 0.27 | 0.52 | 0.38 | 0.56 | 0.56 |
| Hydrocarbon having 4 or more carbon atoms | [kg/hr] | 3.70 | 3.70 | 3.70 | 3.70 | 3.70 | 0.00 |
| Total amount of bypass raw material fed | [kg/hr] | 4.29 | 6.43 | 2.68 | 4.82 | 2.14 | 2.14 |
| Ethanol | [kg/hr] | 4.00 | 6.00 | 2.50 | 4.50 | 2.00 | 2.00 |
| Water | [kg/hr] | 0.29 | 0.43 | 0.18 | 0.32 | 0.14 | 0.14 |
| Total | [kg/hr] | 14.20 | 14.20 | 14.20 | 14.20 | 14.20 | 10.50 |

(continued)

|  |  | Example 1 | Example 2 | Example 3 | Example 4 | Example 5 | Example 6 |
|---|---|---|---|---|---|---|---|
| Reactor of dehydration step |  | Adiabatic | Adiabatic | Adiabatic | Adiabatic | Isothermal | Isothermal |
| Reaction temperature | [°C] | - | - | - | - | 350 | 350 |
| Catalyst bed inlet temperature | [°C] | 480 | 480 | 480 | 480 | - | - |
| Catalyst bed outlet temperature | [°C] | 285 | 309 | 261 | 286 | - | - |
| Lowest internal temperature of reactor | [°C] | 285 | 309 | 261 | 286 | 341 | 329 |
| Pressure | [MPaG] | 0.20 | 0.20 | 0.20 | 0.20 | 0.20 | 0.20 |
| WHSV | [hr⁻¹] | 3.0 | 3.0 | 3.0 | 2.0 | 5.0 | 5.0 |
| Ratio in dehydration reaction gas |  |  |  |  |  |  |  |
| Ethylene | [% by mass] | 30.8% | 27.6% | 34.1% | 30.7% | 39.1% | 54.5% |
| Ethanol | [% by mass] | 3.8% | 0.3% | 2.8% | 1.8% | 0.1% | 0.3% |
| Diethyl ether | [% by mass] | 3.0% | 2.4% | 3.7% | 3.2% | 0.4% | 2.8% |
| Water | [% by mass] | 24.9% | 52.4% | 67.9% | 60.5% | 29.8% | 42.4% |
| Hydrocarbon having 4 or more carbon atoms | [% by mass] | 37.3% | 47.6% | 32.1% | 39.5% | 30.7% | trace |
| Propylene | [% by mass] | 0.2% | 0.1% | trace | 0.2% | trace | trace |
| Ethanol conversion rate | [mol%] | 93.5% | 99.4% | 95.8% | 96.8% | >99.9% | 99.7% |
| Productivity of diethyl ether as by-product | [mol%] | 6.4% | 6.1% | 7.2% | 7.1% | 0.7% | 3.7% |

[Table 3]

Table 3

|  | Example 1 | Example 2 | Example 3 | Example 4 | Example 5 | Example 6 |
|---|---|---|---|---|---|---|
| $\dfrac{EY}{EtOH} - 10 \times \dfrac{Ether}{EtOH + EY + Ether}$ | 0.96 | 0.47 | 2.01 | 0.82 | 3.82 | 3.56 |
| Ethylene/(ethanol + 2 × diethyl et molar ratio ther) | 1.06 | 0.57 | 1.93 | 0.94 | 3.75 | 3.24 |
| Molar ratio of olefin having 4 to 6 atoms/ethylene carbon | 0.26 | 0.37 | 0.20 | 0.27 | 0.17 | 0.17 |
| Olefin conversion catalyst | 2 | 4 | 2 | 5 | 2 | 2 |

(continued)

|  |  | Example 1 | Example 2 | Example 3 | Example 4 | Example 5 | Example 6 |
|---|---|---|---|---|---|---|---|
| Silica/alumina ratio | [-] | 1000 | 280 | 1000 | 1000 | 1000 | 1000 |
| Dope element |  | - | P | - | Ag | - | - |
| Dope element content | [% by mass] | - | 0.18% | - | 0.16% | - | - |
| Amount of raw material mixture fed | [kg/hr] | 14.20 | 14.20 | 14.20 | 14.20 | 14.20 | 14.20 |
| Ethylene | [kg/hr] | 3.08 | 2.16 | 3.93 | 2.89 | 4.71 | 4.56 |
| Ethanol | [kg/hr] | 4.38 | 6.02 | 2.83 | 4.67 | 2.01 | 2.02 |
| Diethyl ether | [kg/hr] | 0.30 | 0.19 | 0.42 | 0.30 | 0.04 | 0.23 |
| Water | [kg/hr] | 2.75 | 2.13 | 3.33 | 2.63 | 3.74 | 3.69 |
| Hydrocarbon having 4 or more carbon atoms | [kg/hr] | 3.70 | 3.70 | 3.70 | 3.70 | 3.70 | 3.70 |
| Pressure WHSV | [MPaG] [hr$^{-1}$] | 0.15 2.0 | 0.15 3.0 | 0.15 2.0 | 0.15 2.0 | 0.15 2.0 | 0.15 2.0 |
| Reaction temperature | [°C] | 540 | 540 | 540 | 540 | 540 | 540 |
| Catalyst bed inlet temperature | [°C] | 534 | 552 | 517 | 538 | 501 | 502 |
| Catalyst bed outlet temperature | [°C] | 546 | 528 | 563 | 542 | 579 | 578 |
| Temperature difference between inlet and outlet | [K] | +12 | -24 | +46 | +4 | +78 | +76 |
| Reactor outlet composition |  |  |  |  |  |  |  |
| Propylene yield | [% by mass] | 20.8 | 19.0 | 22.5 | 20.4 | 24.1 | 24.0 |
| Aromatic yield | [% by mass] | 2.3 | 1.4 | 3.2 | 2.1 | 4.0 | 3.9 |
| Coke yield | [wtppm] | 238 | 256 | 289 | 226 | 340 | 334 |

[Table 4]

Table 4

|  |  | Example 7 | Example 8 | Example 9 | Comparative Example 1 |
|---|---|---|---|---|---|
| Dehydration catalyst |  | 1 | 1 | 2 | 1 |
| Silica/alumina molar ratio | [-] | 40 | 40 | 1000 | 40 |
| Dope element | [-] | - | - | - |  |
| Dope element content | [% by mass] | - | - | - |  |
| Amount of catalyst | [kg] | 0.42 | 0.29 | 1.44 | 1.56 |
| Total amount of dehydration raw material fed | [kg/hr] | 3.54 | 2.46 | 13.99 | 10.50 |
| Ethanol | [kg/hr] | 3.30 | 2.30 | 6.80 | 9.80 |
| Water | [kg/hr] | 0.24 | 0.16 | 7.19 | 0.70 |

(continued)

| | | Example 7 | Example 8 | Example 9 | Comparative Example 1 |
|---|---|---|---|---|---|
| Total amount of bypass raw material fed | [kg/hr] | 6.96 | 8.04 | 3.21 | 0.00 |
| Ethanol | [kg/hr] | 6.50 | 7.50 | 3.00 | 0.00 |
| Water | [kg/hr] | 0.46 | 0.54 | 0.21 | 0.00 |
| Total | [kg/hr] | 10.50 | 10.50 | 17.20 | 10.50 |
| Reactor of dehydration step | | Isothermal | Isothermal | Adiabatic | Isothermal |
| Reaction temperature | [°C] | 350 | 350 | | 350 |
| Catalyst bed inlet temperature | [°C] | | | 480 | |
| Catalyst bed outlet temperature | [°C] | | | 326 | |
| Lowest internal temperature of reactor | [°C] | 331 | 332 | 326 | 332 |
| Pressure | [MPaG] | 0.20 | 0.20 | 0.20 | 0.20 |
| WHSV | [hr$^{-1}$] | 5.0 | 5.0 | 3.0 | 4.0 |
| Ratio in dehydration reaction gas | | | | | |
| Ethylene | [% by mass] | 54.1% | 53.8% | 21.9% | 53.4% |
| Ethanol | [% by mass] | 1.7% | 1.6% | 3.6% | 1.8% |
| Diethyl ether | [% by mass] | 2.3% | 2.7% | 7.3% | 3.0% |
| Water | [% by mass] | 42.0% | 41.9% | 67.2% | 41.8% |
| Propylene | [% by mass] | trace | trace | trace | 0.1% |
| Ethanol conversion rate | [mol%] | 98.2% | 98.3% | 92.5% | 98.1% |
| Productivity of diethyl ether as by-product | [mol%] | 3.0% | 3.6% | 18.6% | 4.0% |

[Table 5]

Table 5

| | | | Example 7 | Example 8 | Example 9 | Comparative Example 1 | Comparative Example 2 |
|---|---|---|---|---|---|---|---|
| $\dfrac{EY}{EtOH} - 10 \times \dfrac{Ether}{EtOH + EY + Ether}$ | | | 0.43 | 0.28 | 0.74 | 48.39 | - |
| Ethylene/(ethanol + 2 × diethyl ether) molar ratio | | | 0.47 | 0.29 | 1.05 | 26.94 | - |
| Olefin conversion catalyst | | | 2 | 6 | 2 | 2 | 2 |
| | Silica/alumina ratio | [-] | 1000 | 1000 | 1000 | 1000 | 1000 |
| | Dope element | [-] | - | - | - | - | - |
| | Dope element content | [% by mass] | - | - | - | - | - |
| Amount of raw material mixture fed | | [kg/hr] | 10.50 | 10.50 | 17.20 | 10.50 | 10.50 |
| | Ethylene | [kg/hr] | 1.91 | 1.35 | 3.06 | 5.62 | 0.0 |
| | Ethanol | [kg/hr] | 6.56 | 7.54 | 3.51 | 0.19 | 9.8 |
| | Diethyl ether | [kg/hr] | 0.08 | 0.03 | 1.02 | 0.31 | 0.0 |
| | Water | [kg/hr] | 1.95 | 1.58 | 9.61 | 4.39 | 0.7 |
| Pressure | | [MPaG] | 0.15 | 0.15 | 0.15 | 0.15 | 0.15 |
| WHSV | | [hr$^{-1}$] | 3.0 | 3.0 | 3.0 | 3.0 | 3.0 |
| Reaction temperature | | [°C] | 540 | 540 | 540 | 540 | 540 |
| Catalyst bed inlet temperature | | [°C] | 531 | 548 | 515 | 419 | 592 |
| Catalyst bed outlet temperature | | [°C] | 549 | 532 | 565 | 661 | 488 |
| Temperature difference between inlet and outlet | | [K] | +18 | -16 | +50 | +242 | -104 |
| Reactor outlet composition | | | | | | | |
| Propylene yield | | [% by mass] | 21.1 | 19.4 | 17.5 | - | 11.4 |
| Aromatic yield | | [% by mass] | 1.7 | 0.8 | 1.4 | - | 0.9 |
| Coke yield | | [wtppm] | 247 | 244 | 295 | - | 376 |

[Table 6]

Table 6

| | | | Example 1 | Example 10 | Example 11 | Example 12 | Comparative Example 3 |
|---|---|---|---|---|---|---|---|
| $\frac{EY}{EtOH} - 10 \times \frac{Ether}{EtOH + EY + Ether}$ | | | 0.96 | 0.74 | 0.70 | 2.96 | -0.79 |
| Ethylene/(ethanol + 2 × diethyl ether) molar ratio | | | 1.06 | 1.06 | 1.06 | 3.24 | 1.06 |
| Molar ratio of olefin having 4 to 6 carbon atoms/ethylene | | | 0.26 | 0.26 | 0.26 | 0.17 | 0.26 |
| Olefin conversion catalyst | | | 2 | 2 | 2 | 2 | 2 |
| | Silica/alumina ratio | [-] | 1000 | 1000 | 1000 | 1000 | 1000 |
| | Dope element | | - | - | - | - | - |
| | Dope element content | [% by mass] | - | - | - | - | - |
| Amount of raw material mixture fed | | [kg/hr] | 14.20 | 14.70 | 14.70 | 14.20 | 18.20 |
| | Ethylene | [kg/hr] | 3.08 | 3.08 | 3.08 | 4.56 | 3.08 |
| | Ethanol | [kg/hr] | 4.38 | 4.38 | 4.38 | 2.02 | 4.38 |
| | Diethyl ether | [kg/hr] | 0.30 | 0.30 | 0.30 | 0.23 | 0.30 |
| | Water | [kg/hr] | 2.75 | 2.75 | 2.75 | 3.69 | 2.75 |
| | Hydrocarbon having 4 or more carbon atoms | [kg/hr] | 3.70 | 3.70 | 3.70 | 3.70 | 3.70 |
| | Ether other than diethyl ether | [kg/hr] | 0.00 | 0.50 | 0.50 | 1.20 | 4.00 |
| Pressure | | [MPaG] | 0.15 | 0.15 | 0.15 | 0.15 | 0.15 |
| WHSV | | [hr-1] | 2.0 | 2.0 | 2.0 | 2.0 | 2.0 |
| Reaction temperature | | [°C] | 540 | 540 | 540 | 540 | - |
| Catalyst bed inlet temperature | | [°C] | 534 | 543 | 539 | 524 | - |
| Catalyst bed outlet temperature | | [°C] | 546 | 537 | 541 | 556 | - |
| Temperature difference between inlet and outlet | | [K] | +12 | -6 | +2 | +32 | - |
| Reactor outlet composition | | | | | | | |
| Propylene yield | | [% by mass] | 20.8 | 19.9 | 20.3 | 21.8 | - |
| Aromatic yield | | [% by mass] | 2.3 | 1.9 | 2.1 | 2.8 | - |

(continued)

|  |  | Example 1 | Example 10 | Example 11 | Example 12 | Comparative Example 3 |
|---|---|---|---|---|---|---|
| Coke yield | [wtppm] | 238 | 229 | 223 | 268 | - |

**Reference Signs List**

1 and 2 ... reactor, 3 ... first distillation column, 4 ... second distillation column, 5 ... third distillation column, 6 ... fourth distillation column, 7 ... fifth distillation column, 8 ... cooler

**Claims**

1.  A method for converting ethanol, comprising:

    a dehydration step of subjecting a dehydration raw material containing ethanol to dehydration reaction by a dehydration catalyst in a reactor to obtain a dehydration reaction gas containing ethylene; and
    an olefin conversion step of contacting a raw material mixture containing the dehydration reaction gas with an olefin conversion catalyst in a reactor to obtain a reaction gas containing an olefin having 3 or more carbon atoms, wherein
    the raw material mixture contains an ether, and
    in the raw material mixture, a value represented by the following expression (1):

$$\frac{EY}{EtOH} - 10 \times \frac{Ether}{EtOH + EY + Ether} \cdots \ (1)$$

    wherein EY represents a molar quantity of ethylene contained in the raw material mixture, EtOH represents a molar quantity of ethanol contained in the raw material mixture, and Ether represents a molar quantity of the ether contained in the raw material mixture
    is 0.20 to 4.0.

2.  The method for converting ethanol according to claim 1, wherein the value represented by the expression (1) in the raw material mixture is 0.40 to 3.0.

3.  The method for converting ethanol according to claim 1, wherein the ether comprises at least one selected from the group consisting of diethyl ether, methyl tertiary butyl ether, and ethyl tertiary butyl ether.

4.  The method for converting ethanol according to claim 1, wherein a content of the ether in the raw material mixture is 20% by mass or less.

5.  A method for converting ethanol, comprising:

    a dehydration step of subjecting a dehydration raw material containing ethanol to dehydration reaction by a dehydration catalyst in an amount of 10 parts by mass or more with respect to 100 parts by mass of the ethanol fed per hour in a reactor to obtain a dehydration reaction gas containing ethylene; and
    an olefin conversion step of contacting a raw material mixture containing the dehydration reaction gas with an olefin conversion catalyst in a reactor to obtain a reaction gas containing an olefin having 3 or more carbon atoms.

6.  The method for converting ethanol according to claim 5, wherein in the dehydration step, a conversion rate of ethanol in the dehydration raw material is brought to 90% by mol or more.

7.  A method for converting ethanol, comprising:

    a dehydration step of subjecting a dehydration raw material containing ethanol to dehydration reaction by a dehydration catalyst in a reactor and bringing a conversion rate of ethanol in the dehydration raw material to 90% by mol or more to obtain a dehydration reaction gas containing ethylene; and

an olefin conversion step of feeding ethanol and the dehydration reaction gas into a reactor and contacting the raw material mixture with an olefin conversion catalyst in the reactor to obtain a reaction gas containing an olefin having 3 or more carbon atoms.

8. The method for converting ethanol according to claim 7, wherein a weight ratio E1/E2 of ethanol (E1) to be fed into the reactor in the dehydration step to ethanol (E2) to be fed into the reactor in the olefin conversion step is 0.10 to 4.0.

9. The method for converting ethanol according to any one of claims 1 to 8, wherein a content of propylene in the dehydration reaction gas is 20% by mass or less.

10. The method for converting ethanol according to any one of claims 1 to 8, wherein a content of diethyl ether in the raw material mixture is 10% by mass or less.

11. The method for converting ethanol according to any one of claims 1 to 8, wherein the raw material mixture contains a hydrocarbon having 4 to 6 carbon atoms.

12. The method for converting ethanol according to any one of claims 1 to 8, wherein a molar ratio of olefin having 4 to 6 carbon atoms/ethylene in the raw material mixture is 3.5 or less.

13. The method for converting ethanol according to any one of claims 1 to 8, wherein the reactor in the olefin conversion step is an adiabatic reactor.

14. The method for converting ethanol according to claim 13, wherein the adiabatic reactor in the olefin conversion step is a fixed-bed single-stage adiabatic reactor.

15. The method for converting ethanol according to any one of claims 1 to 8, further comprising
a separation step of separating the reaction gas into fraction A mainly comprising a hydrocarbon having 1 to 3 carbon atoms and fraction B mainly comprising a hydrocarbon having 4 to 6 carbon atoms.

16. The method for converting ethanol according to any one of claims 1 to 8, further comprising:
a C1 removal step of separating the fraction A into fraction C mainly comprising a hydrocarbon having 1 carbon atom and fraction D mainly comprising a hydrocarbon having 2 to 3 carbon atoms:

a C2 removal step of separating the fraction D into fraction E mainly comprising a hydrocarbon having 2 carbon atoms and fraction F mainly comprising a hydrocarbon having 3 carbon atoms;
an ethylene purification step of separating the fraction E into fraction G mainly comprising ethylene and fraction H mainly comprising ethane; and
a propylene purification step of separating the fraction F into fraction I mainly comprising propylene and fraction J mainly comprising propane.

17. The method for converting ethanol according to any one of claims 1 to 8, wherein the dehydration catalyst and/or the olefin conversion catalyst is a solid acidic catalyst.

18. The method for converting ethanol according to claim 17, wherein the solid acidic catalyst is a zeolite-containing catalyst.

19. The method for converting ethanol according to claim 17, wherein a silica/alumina molar ratio of zeolite in the zeolite-containing catalyst is 20 to 2000.

20. The method for converting ethanol according to claim 17, wherein the zeolite-containing catalyst comprises at least one element selected from the group consisting of phosphorus and an element belonging to group 11 of the periodic table.

21. A method for producing a hydrocarbon, comprising:

a dehydration step of subjecting a dehydration raw material containing ethanol to dehydration reaction by a dehydration catalyst in a reactor to obtain a dehydration reaction gas containing ethylene; and
an olefin conversion step of contacting a raw material mixture containing the dehydration reaction gas with an olefin conversion catalyst in a reactor to obtain a reaction gas containing an olefin having 3 or more carbon atoms, wherein
the raw material mixture contains an ether, and
in the raw material mixture, a value represented by the following expression (1):

$$\frac{EY}{EtOH} - 10 \times \frac{Ether}{EtOH + EY + Ether} \cdots \quad (1)$$

wherein EY represents a molar quantity of ethylene contained in the raw material mixture, EtOH represents a molar quantity of ethanol contained in the raw material mixture, and Ether represents a molar quantity of the ether contained in the raw material mixture
is 0.20 to 4.0.

22. A method for producing propylene, comprising
a propylene separation step of separating a fraction mainly comprising propylene from a reaction gas obtained by the method for converting ethanol according to any one of claims 1 to 8.

23. A method for producing an aromatic compound, comprising
an aromatic compound separation step of separating a fraction mainly comprising an aromatic compound from a reaction gas obtained by the method for converting ethanol according to any one of claims 1 to 8.

24. An apparatus for converting ethanol, comprising:

a reactor which subjects a dehydration raw material containing ethanol to dehydration reaction by a dehydration catalyst to obtain a dehydration reaction gas containing ethylene; and
a reactor which contacts a raw material mixture containing the dehydration reaction gas with an olefin conversion catalyst to obtain a reaction gas containing an olefin having 3 or more carbon atoms.

25. The apparatus for converting ethanol according to claim 24, further comprising
a separation apparatus which separates the reaction gas into fraction A mainly comprising a hydrocarbon having 1 to 3 carbon atoms and fraction B mainly comprising a hydrocarbon having 4 to 6 carbon atoms.

26. The apparatus for converting ethanol according to claim 24, further comprising:

an ethanol feed line which introduces ethanol to the reactor for obtaining the reaction gas; and
a dehydration reaction gas feed line which introduces the dehydration reaction gas to the reactor for obtaining the reaction gas.

[Figure 1]

[Figure 2]

[Figure 3]

[Figure 4]

[Figure 5]

[Figure 6]

**EP 4 596 525 A1**

## INTERNATIONAL SEARCH REPORT

| International application No. |
|---|
| **PCT/JP2023/027601** |

| A. | CLASSIFICATION OF SUBJECT MATTER |
|---|---|

*C07C 6/04*(2006.01)i; *B01D 3/14*(2006.01)i; *B01J 8/02*(2006.01)i; *B01J 29/40*(2006.01)i; *B01J 29/44*(2006.01)i;
*C07B 61/00*(2006.01)i; *C07C 1/24*(2006.01)i; *C07C 11/06*(2006.01)i
FI: C07C6/04; C07C11/06; C07C1/24; B01J29/40 M; B01J29/44 M; B01J8/02 Z; C07B61/00 300; B01D3/14 A

According to International Patent Classification (IPC) or to both national classification and IPC

| B. | FIELDS SEARCHED |
|---|---|

Minimum documentation searched (classification system followed by classification symbols)

C07C6/04; B01D3/14; B01J8/02; B01J29/40; B01J29/44; C07B61/00; C07C1/24; C07C11/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2013-209282 A (TOSOH CORP) 10 October 2013 (2013-10-10)<br>claims, examples, tables, etc. | 1-26 |
| A | JP 2007-291076 A (MITSUBISHI CHEMICALS CORP) 08 November 2007 (2007-11-08)<br>claims, examples, tables, etc. | 1-26 |
| A | JP 2007-290991 A (IDEMITSU KOSAN CO LTD) 08 November 2007 (2007-11-08)<br>claims, examples, tables, etc. | 1-26 |
| A | US 2013/0245290 A1 (SHELL OIL COMPANY) 19 September 2013 (2013-09-19)<br>claims, examples | 1-26 |
| E, A | WO 2023/145941 A1 (ASAHI CHEMICAL IND) 03 August 2023 (2023-08-03)<br>claims, examples, figures, etc. | 1-26 |

☐ Further documents are listed in the continuation of Box C.   ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **04 October 2023** | **24 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

40

## INTERNATIONAL SEARCH REPORT
### Information on patent family members

International application No.

**PCT/JP2023/027601**

| Patent document cited in search report | | | Publication date (day/month/year) | Patent family member(s) | | | Publication date (day/month/year) |
|---|---|---|---|---|---|---|---|
| JP | 2013-209282 | A | 10 October 2013 | (Family: none) | | | |
| JP | 2007-291076 | A | 08 November 2007 | WO | 2007/114195 | A1 | |
| | | | | claims, examples, tables | | | |
| | | | | KR 10-2008-0114763 | | A | |
| | | | | CN | 101410353 | A | |
| JP | 2007-290991 | A | 08 November 2007 | (Family: none) | | | |
| US | 2013/0245290 | A1 | 19 September 2013 | WO | 2013/034678 | A1 | |
| | | | | claims, examples | | | |
| | | | | CN | 103796975 | A | |
| WO | 2023/145941 | A1 | 03 August 2023 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014025021 A
- WO 2015029355 A
- CN 110560155
- US 20140018593
- CN 110871107
- JP 5426983 B

**Non-patent literature cited in the description**

- Adiabatic Fixed-Bed Reactors. Elsevier, 2014, vol. 1, 5-24
- *Stud. Surf. Sci. Catal.*, 1987, vol. 33, 167-215
- *Chemical Reviews*, 2003, vol. 103, 663-702